# EUROPEAN PATENT APPLICATION

(11) **EP 4 847 622 A1**
(43) Date of publication of application: **02.12.2026**
(21) Application number: 25745196.3
(22) Date of filing: 23.01.2025
(51) Int. Cl.: C12N 15/86, C12N 1/00, C12N 5/10, C12N 7/01, C12N 9/99, C12N 15/864, C12N 15/90

(54) **METHOD FOR PRODUCING VIRAL VECTOR**

(30) Priority: 24.01.2024 JP 2024008571
(71) Applicant: FUJIFILM Corporation, Tokyo 106-8620 (JP)
(72) Inventor: MORI Yusuke, Ashigarakami-gun, Kanagawa 258-8577 (JP); EDO Michiko, Ashigarakami-gun, Kanagawa 258-8577 (JP); TONOIKE Nozomu, Ashigarakami-gun, Kanagawa 258-8577 (JP); MURAGUCHI Taichi, Ashigarakami-gun, Kanagawa 258-8577 (JP)
(74) Representative: Hoffmann Eitle
(86) International application number: PCT/JP2025/002045
(87) International publication number: WO 2025/159153

(57) **Abstract**

An object of the present invention is to provide a method for manufacturing a viral vector, in which a production amount of a viral vector can be improved. According to the present invention, there is provided a method for producing a viral vector, the method including culturing producer cells or packaging cells in a culture solution containing the producer cells or the packaging cells at a cell density of 1 × 10⁶ cells/mL or more and a histone deacetylase inhibitor.

## Description

### Technical Field

The present invention relates to a method for producing a viral vector, the method including culturing a producer cell or a packaging cell in a predetermined state.

### Background Art

The adeno-associated virus (AAV) is a linear single-stranded DNA virus belonging to Parvoviridae. The wild-type AAV genome contains a replication regulatory gene (Rep gene) and a capsid structural gene (Cap gene), and inverted terminal repeat sequences (ITR) are adjacent to these genes for viral replication and packaging. The AAV vector can transfer a gene to proliferating and non-proliferating cells, and can be expressed for a long term particularly in non-dividing cells. In addition, AAV is considered to be non-pathogenic and has low immunogenicity. From the above, the AAV vector has been clinically applied as a vector for gene therapy. In general, an AAV vector for gene therapy encapsulates any therapeutic gene flanked by ITRs. As a method for producing a viral vector such as an AAV vector, production of the viral vector by culturing producer cells or packaging cells has been carried out.

Patent Document 1 describes that rAAV particles are generated by culturing cells capable of generating recombinant AAV (rAAV) particles in the presence of a histone deacetylase (HDAC) inhibitor. In Non-Patent Document 1, it is shown that the titer of the rAAV8 genome is improved by using M344 (a selective HDAC inhibitor) in a case of producing rAAV using HEK293 cells.

### Prior Art Documents

### Patent Documents

Patent Document 1: WO2021/533757A

### Non-Patent Documents

Non-Patent Document 1: Joseph M. Scarrott et al., Biotechnol. J. 2023: 18: 2200450

### Summary of Invention

### Object to be solved by the invention

Currently, a gene therapy agent using AAV vectors have been put on the market, and there is a demand for the development of a production method that improves the production amount of viral vectors such as AAV vectors. An object to be achieved of the present invention is to provide a method of producing a viral vector, the method being capable of improving the production amount of the viral vectors.

### Means for solving the object

As a result of intensive studies to achieve the above-described object, the present inventors have found that the production amount of a viral vector can be improved by culturing producer cells or packaging cells at a cell density of 1 × 10⁶ cells/mL or more in the presence of a histone deacetylase inhibitor. The present invention has been completed based on these findings.

According to an aspect of the present invention, the following inventions are provided.
<1> A method for producing a viral vector, the method comprising: culturing producer cells or packaging cells in a culture solution containing the producer cells or the packaging cells at a cell density of 1 × 10⁶ cells/mL or more and a histone deacetylase inhibitor.
<2> The production method according to <1>, in which the histone deacetylase inhibitor is a compound having a benzamide structure or a hydroxamic acid.
<3> The production method according to <1>, in which the histone deacetylase inhibitor is a compound represented by Formula (1) or Formula (2),

   R¹-CONH-R² (1)

   in Formula (1), one of R¹ or R² is an aromatic group which may have a substituent, and the other of R¹ or R² is an amino group which may have a substituent, an alkyl group which may have a substituent, an alkenyl group which may have a substituent, an aromatic group which may have a substituent, or an alkoxy group which may have a substituent,

      R³-CONH-OH (2)
   in Formula (2), R³ is an alkyl group which may have a substituent or an alkenyl group which may have a substituent.
<4> The production method according to <1>, in which the histone deacetylase inhibitor is a compound represented by Formula (1A), Formula (1B), or Formula (2A), 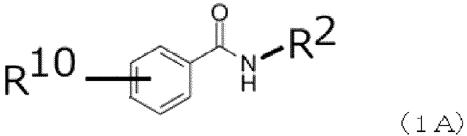
   in Formula (1A), R¹⁰ represents halogen, a heterocyclic group which may have a substituent, an alkyl group having 1 to 6 carbon atoms which may have a substituent, or an amino group which may have a substituent, and R² is an amino group which may have a substituent, an alkyl group which may have a substituent, or an aromatic group which may have a substituent, 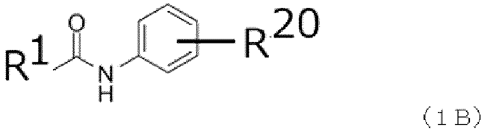
   in Formula (1B), R²⁰ represents an amino group, and R¹ is an alkyl group which may have a substituent or an aromatic group which may have a substituent,

      R³¹-R³⁰-CONH-OH (2A)
   in Formula (2A), R³⁰ represents -(CH₂)ₙ- or CH=CH-, n represents an integer of 2 to 8, and R³¹ represents an aromatic group which may have a substituent or CONH-OH.
<5> The production method according to <4>, in which in Formula (1A), R¹⁰ is bromine, 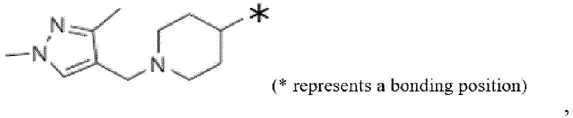 methyl, 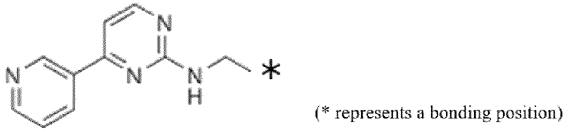 , or

   CH₃-CO-NH-.
<6> The production method according to <1>, in which the histone deacetylase inhibitor is any one of the following compounds, 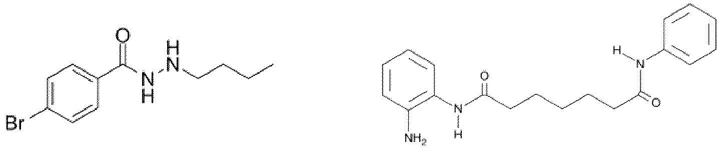 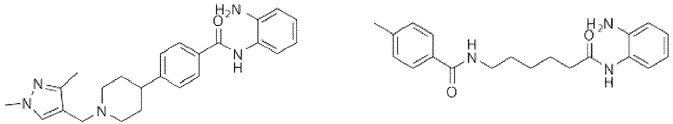 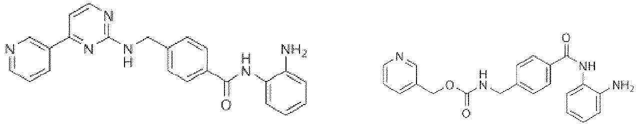 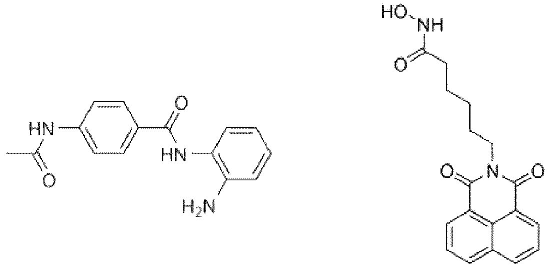 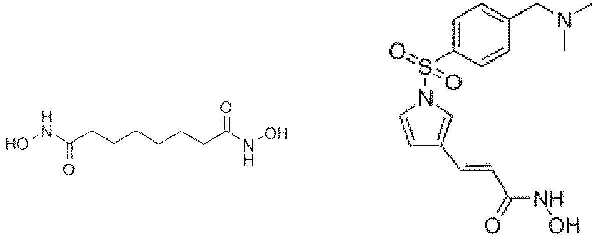 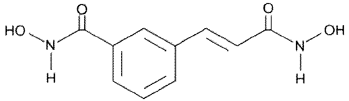
<7> The production method according to any one of <1> to <6>, further comprising: culturing the cells by perfusion culture.
<8> The production method according to any one of <1> to <6>, in which a virus is an adeno-associated virus.
<9> The production method according to <8>, in which the producer cells or the packaging cells are cells in which a Rep gene, a Cap gene, and a helper gene are incorporated into a chromosome.
<10> The production method according to any one of <1> to <6>, in which the cells are animal cells.
<11> The production method according to any one of <1> to <6>, in which a content of the histone deacetylase inhibitor in the culture solution is 50 µmol/L or less.
<12> The production method according to any one of <1> to <6>, in which the culture is performed for 24 hours or more in the culture solution containing the histone deacetylase inhibitor.
<13> The production method according to any one of <1> to <6>, in which the cell density is 30 × 10⁶ cells/mL or more.
<14> The production method according to any one of <1> to <6>, in which the culture is a suspension culture.

### Effect of the invention

According to the present invention, it is possible to improve the production amount of the viral vector.

### BRIEF DESCRIPTION OF THE DRAWINGS

[FIG. 1]FIG. 1 shows a titer improvement effect of a compound.
[FIG. 2] FIG. 2 shows results of measurement of ddPCR titer in high-density cells.
[FIG. 3] FIG. 3 shows results of measurement of a cell density in a reactor.
[FIG. 4] FIG. 4 shows results of measurement of an AAV titer in a cell culture solution continuously collected for 7 days.
[FIG. 5] FIG. 5 shows results of high-performance chromatography measurement.
[FIG. 6] FIG. 6 shows cell culture density and the titer improvement effect of the compound.
[FIG. 7] FIG. 7 shows a titer improvement effect at different concentrations of the compound.

### Embodiments for carrying out the invention

Hereinafter, an example of an embodiment of the present disclosure will be described. However, the present disclosure is not limited to the following embodiments, and can be implemented with appropriate changes within the scope of the object of the present disclosure. In the present specification, a numerical value range indicated using "to" means a range including numerical values described before and after "to" as a minimum value and a maximum value, respectively.

### <Terminology>

A promoter means a DNA regulatory region/sequence to which an RNA polymerase can be bound and which is involved in the initiation of transcription of a downstream coding sequence or a downstream non-coding sequence.

A vector is a DNA or RNA molecule used for artificially transporting an exogenous gene into another cell. A vector for expressing a gene is referred to as an expression vector. In a case where a vector containing an exogenous gene to be introduced is introduced into a cell, the exogenous gene is replicated and/or expressed in the cell. Examples of the vector include an episomal (for example, plasmid) vector and a non-episomal vector. The vector can be introduced into a host cell by methods such as transfection, transduction, cell fusion, and lipofection.

In the present specification, a gene means a nucleic acid molecule encoding a polypeptide or RNA, and examples thereof include a nucleic acid molecule such as cDNA or genomic DNA.

A method for producing a viral vector according to the present embodiment of the present invention includes culturing producer cells or packaging cells in a culture solution containing the producer cells or the packaging cells at a cell density of 1 × 10⁶ cells/mL or more and a histone deacetylase inhibitor.

In the present invention, it is speculated that in a producer cell or a packaging cell in which a gene necessary for expression of a viral vector or a part thereof is inserted into a chromosome of a host cell, the activity of a histone deacetylase (HDAC) expressed in the cell is inhibited, whereby histone acetylation is enhanced to induce a change in chromatin structure, and thus the titer and the full ratio (the ratio of the amount of capsid containing a full-length gene to the total amount of capsid) of the virus vector can be improved.

The histone deacetylase inhibitor is not particularly limited as long as it is a substance having an action of inhibiting a histone deacetylase, and it is preferably a compound having a benzamide structure or a hydroxamic acid.

Preferred examples of the histone deacetylase inhibitor include a compound represented by Formula (1) or Formula (2).

R¹-CONH-R² (1)

In Formula (1), one of R¹ or R² is an aromatic group which may have a substituent, and the other of R¹ or R² is an amino group which may have a substituent, an alkyl group which may have a substituent, an alkenyl group which may have a substituent, an aromatic group which may have a substituent, or an alkoxy group which may have a substituent.

R³-CONH-OH (2)

In Formula (2), R³ is an alkyl group which may have a substituent or an alkenyl group which may have a substituent.

More preferred examples of the histone deacetylase inhibitor include a compound represented by Formula (1A), Formula (1B), or Formula (2A). 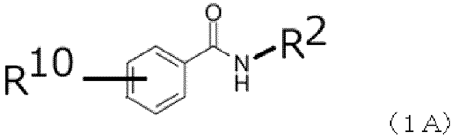 in Formula (1A), R¹⁰ represents halogen, a heterocyclic group which may have a substituent, an alkyl group having 1 to 6 carbon atoms which may have a substituent, or an amino group which may have a substituent, and R² is an amino group which may have a substituent, an alkyl group which may have a substituent, or an aromatic group which may have a substituent,

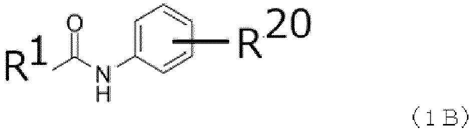

In Formula (1B), R²⁰ represents an amino group, and R¹ is an alkyl group which may have a substituent or an aromatic group which may have a substituent.

R³¹-R³⁰-CONH-OH (2A)

In Formula (2A), R³⁰ represents -(CH₂)ₙ- or CH=CH-, n represents an integer of 2 to 8, and R³¹ represents an aromatic group which may have a substituent or CONH-OH.

The aromatic group is preferably a phenyl group.

The alkyl group is preferably an alkyl group having 1 to 8 carbon atoms and more preferably an alkyl group having 1 to 6 carbon atoms. The alkyl group may be linear or branched.

The alkenyl group is preferably an alkenyl group having 2 to 8 carbon atoms, more preferably an alkenyl group having 2 to 6 carbon atoms, and still more preferably an alkenyl group having 2 to 4 carbon atoms.

The alkoxy group is preferably an alkoxy group having 1 to 8 carbon atoms and more preferably an alkoxy group having 1 to 6 carbon atoms. The alkoxy group may be linear or branched.

The halogen is preferably fluorine, chlorine, bromine, or iodine.

Examples of the substituent include a halogen (for example, fluorine, chlorine, bromine, or iodine), an alkyl group having 1 to 6 carbon atoms, -NH₂, -CONHC₆H₅, -CONHC₆H₅(NH₂), -NHCOCH₃, -NHCOC₆H₅(CH₃), -CONHOH, a pyridyl group, an aromatic group substituted with -CONHOH (for example, a phenyl group substituted with -CONHOH), a heterocyclic group which may be substituted, 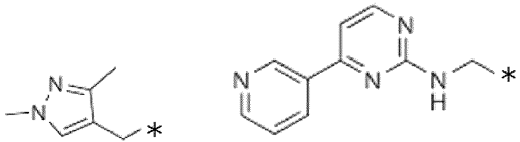 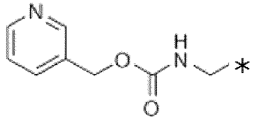 (* represents a bonding position), and the like.

Examples of the heterocyclic group which may be substituted include 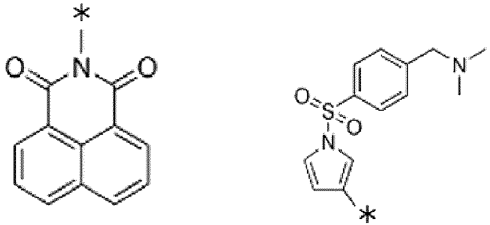 (* represents a bonding position), and the like.

Particularly preferably, in Formula (1A), R¹⁰ represents bromine, 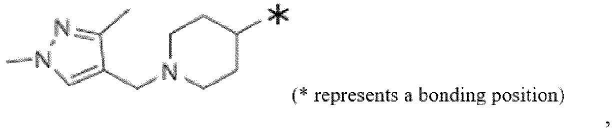 methyl, 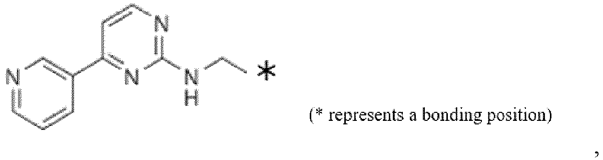 or

CH₃-CO-NH-.

Specific examples of the histone deacetylase inhibitor can include any of the following compounds.

| | |
|---|---|
| UF010 | PAOA |
| 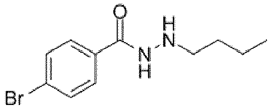 | 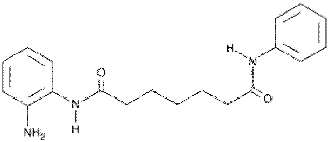 |
| CXD101 | RGFP109 |
| 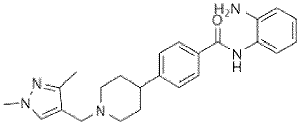 | 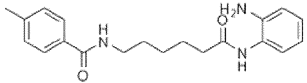 |
| Mocetinostat | MS-275 |
| 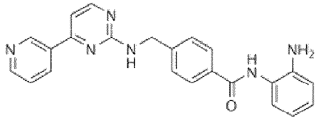 | 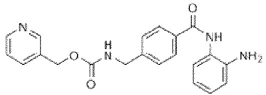 |
| CI-994 | Scriptaid |
| 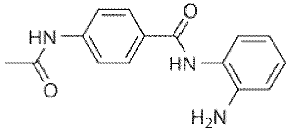 | 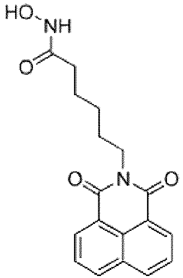 |
| Suberohydroxamic Acid | Resminostat |
| 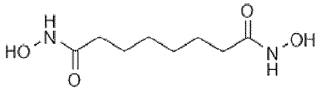 | 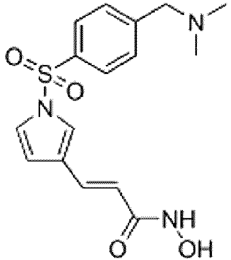 |
| CBHB | |
| 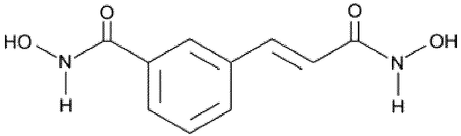 | |

Preferred specific examples of the histone deacetylase inhibitor include UF010, CI-994, and Resminostat.

The content of the histone deacetylase inhibitor in the culture solution is preferably 50 µmol/L or less, more preferably 0.01 µmol/L to 50 µmol/L, still more preferably 0.05 µmol/L to 50 µmol/L, even more preferably 0.1 µmol/L to 50 µmol/L, even still more preferably 1 µmol/L to 20 µmol/L, yet more preferably 1 µmol/L to 10 µmol/L, even yet more preferably 3 µmol/L to 10 µmol/L, and particularly preferably 5 µmol/L to 10 µmol/L.

In the present invention, the culture is preferably performed for 24 hours or more, more preferably performed for 24 hours or more and 240 hours or less, still more preferably performed for 24 hours or more and 120 hours or less, and particularly preferably performed for 48 hours or more and 96 hours or less in the culture solution containing the histone deacetylase inhibitor. The upper limit of the period in which the culture medium contains the histone deacetylase inhibitor is not particularly limited, but is generally 240 hours or less, preferably 120 hours or less, and particularly preferably 96 hours or less.

In a case where a doxycycline-inducing promoter is used for expressing the exogenous recombinant nucleic acid, the culture medium may contain doxycycline. The concentration of doxycycline is preferably 5 µg/mL or less, more preferably 1 µg/mL or less, still more preferably 700 ng/mL or less, and particularly preferably 500 ng/mL or less.

The addition time of doxycycline is preferably 1 hour or more, more preferably 6 hours or more, still more preferably 12 hours or more, even still more preferably 24 hours or more, and yet even still more preferably 48 hours or more.

In the present invention, it is preferable that the culture medium is supplied during the culture, and the supplied culture medium may contain doxycycline and the histone deacetylase inhibitor.
The supplied culture medium is a culture medium to be added during cell culturing. The culture method using the supplied culture medium is not particularly limited, but perfusion culture is desirable. That is, the method according to the embodiment of the present invention preferably includes perfusion culture of the cells.

The period (treatment time) in which doxycycline is allowed to act is preferably 1 hour or more, more preferably 6 hours or more, still more preferably 12 hours or more, even more preferably 24 hours or more, and even still more preferably 48 hours or more.

In the present invention, a producer cell or a packaging cell is used.

The producer cell is a cell having all the genes required for virus production. As the producer cell, it is preferable to use a cell in which all the required genes including the target gene (GOI) encapsulated in the viral vector are incorporated into the chromosome.

The packaging cell is a cell having a part of the genes required for virus production. As the packaging cell, it is preferable to use a cell in which a part of the genes required for virus production is incorporated into the chromosome. The virus can be produced by introducing the remaining genes required for virus production into the packaging cell having a part of the genes required for virus production by transfection.

Examples of the virus include an adeno-associated virus, a lentivirus, a baculovirus, and a retrovirus, and among these, an adeno-associated virus is preferable.

Adeno-associated virus (AAV) refers to a small, incompletely replicative, non-enveloped virus containing single-stranded DNA having about 4,700 bases, belonging to the family Parvoviridae and the genus Dependoparvovirus. It is known that there are more than 100 serum types in AAV, and that the host range and the characteristics of the virus differ depending on the difference in the serum types. Serum type 2 (AAV2) is one of the serum types that have been widely studied for a long time, and it is known that the host range is very wide. The serum type 1 (AAV1), the serum type 5 (AAV5), and the serum type 6 (AAV6) are serum types having higher tissue tropisms. It is said that AAV1 has high gene transfer efficiency into muscle, liver, airway, central nervous system, and the like, AAV5 has high gene transfer efficiency into central nervous system, liver, retina, and the like, and AAV6 has high gene transfer efficiency into heart, muscle, liver, and the like. In the present invention, it is preferable to use serum type 2 or serum type 5. The serum type 5 is particularly preferable.

The adeno-associated virus gene refers to a gene composed of one or a plurality of nucleic acid sequences derived from the serum types of one or a plurality of adeno-associated viruses. The adeno-associated virus gene is preferably a gene involved in replication and packaging of AAV and a gene encoding an AAV constituent protein.

AAV is a non-enveloped virus that proliferates in the presence of helper viruses such as adenovirus and herpesvirus. In the preparation of AAV to be used for gene therapy or nucleic acid introduction, classically, AAV replication has been performed by co-infecting host cells with an adenovirus. In addition, a gene responsible for the helper action of adenovirus has been clarified, and a plasmid incorporating this gene has also been used. For example, a plasmid containing a Rep gene and a Cap gene, an adenovirus helper plasmid, and a plasmid containing a therapeutic or prophylactic gene can be simultaneously transfected into a cell to be packaged into a recombinant AAV (rAAV).

The Rep gene and the Cap gene encode proteins involved in the replication and packaging of the virion. In the wild type, the Rep gene is expressed from a P5 promoter and a P19 promoter. The Cap region expresses VP1, VP2, and VP3. Examples of the promoter naturally retained by the Cap gene include a P40 promoter.

The adeno-associated virus gene (such as a Rep gene and a Cap gene) may be a wild-type gene, but a gene in which modifications such as substitution, deletion, insertion, or addition of bases have been made to the wild-type gene may be used as long as it exhibits an original function.

In a case where a modification such as base substitution, deletion, insertion, or addition is made to the wild-type adeno-associated virus gene (the Rep gene, the Cap gene, or the like), the number of bases to be modified is preferably 1 to 20, more preferably 1 to 10, and still more preferably 1 to 3. The modified base sequence of the adeno-associated virus gene exhibits preferably 85% or more sequence identity, more preferably 90% or more sequence identity, still more preferably 95% or more sequence identity, and even still more preferably 98% or more sequence identity with the base sequence of the wild-type adeno-associated virus gene.

In a case where the Rep gene and the Cap gene are introduced into a cell, a vector containing the Rep gene and the Cap gene can be introduced into the cell. The arrangement and the orientation of the Rep gene and the Cap gene in the vector are not particularly limited, but the orientation of the sequence of the Rep gene and the Cap gene is preferably different.

The Rep gene means a region of the AAV genome encoding a virus replication protein that is known to those skilled in the art and that is collectively required for replication of a virus genome, or its functional homolog, for example, a human herpesvirus 6 (HHV-6) Rep gene (mediating AAV-2 DNA replication is known). Accordingly, the coding region of the Rep gene includes at least a gene encoding REP78 and REP68 (long form of REP protein) and REP52 and REP40 (short form of REP protein) of AAV, or a functional homologue thereof. The coding region of the Rep gene used in the present invention may be derived from any AAV serum type, but is preferably derived from AAV2. Examples of those derived from AAV2 include REP78 and REP68, and REP52 and REP40, and ITR.

The Cap gene means a region in the AAV genome encoding a capsid protein of a virus known to those skilled in the art. Examples of these capsid proteins are AAV capsid proteins VP1, VP2 and VP3. The Cap gene used in the present invention may be derived from any AAV serum type, but is preferably derived from AAV2 or AAV5. AAV5 is particularly preferable.

As the vector containing the Rep gene and the Cap gene, for example, a plasmid, a nucleic acid sequence derived from a virus, or an artificially designed nucleic acid can be used, and a plasmid is preferable.

In the present invention, a target gene (GOI) may be introduced into a cell. It is preferable that the GOI is introduced in a state of being sandwiched between ITRs. The GOI is preferably a therapeutic or prophylactic gene.

As the therapeutic or prophylactic gene, a gene that is incomplete or lost in the genome of the target cell, a gene encoding non-natural protein having a desired biological or therapeutic effect (for example, antiviral function), or the like can be used, but the gene is not particularly limited. Specific examples of the gene for treatment or prevention include a gene used for treatment or prevention of inflammatory diseases, autoimmunity, chronic and infectious diseases (including disorders such as AIDS, cancer, nervous system diseases, cardiovascular diseases, and hypercholesterolemia), various blood diseases such as anemia and hemophilia, or gene deficiency (for example, cystic fibrosis, Gaucher's disease, adenosine deaminase (ADA) deficiency, emphysema, and the like).

The gene for treatment or prevention may be several antisense oligonucleotides (for example, a short-chain oligonucleotide complementary to a sequence around a translation initiation site (AUG codon) of mRNA) that are useful in antisense therapy against cancer and viral disease.

The GOI may be linked to a promoter for expressing the GOI. The promoter for expressing the GOI is not particularly limited, but examples thereof can include a cytomegalovirus-derived promoter (including an enhancer as desired), an SV40 early promoter, a human elongation factor-1α (EF-1α) promoter, a human ubiquitin C promoter, a retrovirus Rous sarcoma virus LTR promoter, a dihydrofolate reductase promoter, a β-actin promoter, and a phosphoglycerate kinase (PGK) promoter. It is preferable that the GOI and the promoter for expressing the gene are sandwiched between ITR sequences.

In the present invention, it is preferable that a virus helper gene derived from an adenovirus is transferred into cells. The virus helper gene is a non-adeno-associated virus gene for enabling replication and packaging of an adeno-associated virus. As the virus helper gene, a gene derived from a virus of a different species other than the adeno-associated virus is used. Specific examples of the virus helper gene include a virus helper gene derived from an adenovirus or a herpesvirus, and the virus helper gene is preferably derived from an adenovirus.

Examples of the virus helper gene derived from adenovirus can include E1A, E1B, E2A, E4, and VA-RNA. In the host cell having all or a part of the E1 region, the region of the adenovirus genome required for replicating the AAV genome and packaging of the AAV genome into the capsid to form a virus virion is the E2A region, the E4 region, and the VA-RNA region. For the function by the E4 region, the 34 kDa E4 protein encoded by the open reading frame 6 (E4ORF6) of the E4 region is required for replicating the AAV. Preferably, the virus helper gene is an E2 gene, an E4 gene, and a VA-RNA gene. More preferably, the virus helper gene is an E2 gene and an E4 gene. The VA-RNA gene is preferably a VA-RNAI gene.

The adenovirus-derived virus helper gene (an E1A, an E1B, an E2A, an E4, a VA-RNA, and the like) may be a wild-type gene, but a gene in which modifications such as substitution, deletion, insertion, addition, or the like of a base have been made to the wild-type gene may be used as long as it exhibits an inherent function.

In a case where modifications such as substitution, deletion, insertion, addition or the like of a base is made to the wild-type virus helper gene, the number of the modified bases is preferably 1 to 20, more preferably 1 to 10, and still more preferably 1 to 3. The modified base sequence of the virus helper gene exhibits preferably 85% or more sequence identity, more preferably 90% or more sequence identity, still more preferably 95% or more sequence identity, and even still more preferably 98% or more sequence identity with the base sequence of the wild-type virus helper gene.

In a case of transferring a virus helper gene into a cell, a vector containing the virus helper gene can be transferred into the cell.

As the vector containing a virus helper gene, for example, a plasmid, a virus-derived sequence, an artificially designed nucleic acid, or the like can be used, and a plasmid is preferable.

The virus helper gene is preferably under the control of a promoter and may be under the control of a promoter capable of regulating expression.

As the promoter capable of regulating expression, a promoter capable of regulating expression on and off by the presence or absence of a stimulus that induces expression in a condition-specific manner can be used. Examples of the stimulus that induces expression in a condition-specific manner include a chemical stimulus, a physical stimulus, and the like, but are not particularly limited. Examples of the chemical stimulus include endogenous hormones, stress responses, lactose, tetracycline or a derivative thereof (for example, doxycycline), cumic acid, rapamycin, FKCsA, abscisic acid (ABA), tamoxifen/Cre-loxP (system in which a Cre promoter modified to be able to induce activation by tamoxifen is used), a riboswitch, and the like. Examples of the physical stimulus include blue light, heat, and the like.

Among the above, the promoter capable of regulating expression is preferably a promoter capable of regulating expression by a drug. The drug is preferably a tetracycline-based drug and more preferably doxycycline. That is, the stimulus that induces the expression in a condition-specific manner may be that the culture medium contains doxycycline.

Specifically, examples of the promoter capable of regulating expression include a tetracycline-responsive promoter, a RU486-inductive promoter, an ecdysone-inductive promoter, a rapamycin-inductive promoter, and a metallothionein promoter. Specific examples of the tetracycline-responsive promoter include a Tet-on/off system (manufactured by TET systems GmbH & Co. KG).

The promoter capable of regulating expression may be a Tet-on/off system which is a promoter capable of regulating expression by tetracycline, or a Tet-on system.

In the Tet on system, the promoter additionally contains at least one Tet operon. By using a Tet operon (tetracycline-controlled transcriptional activation), transcription can be reversibly switched on or off in the presence of one of the tetracycline which is an antibiotic, or a derivative thereof (for example, doxycycline). The Tet repressor protein present in the cell blocks expression by binding to a Tet operator sequence introduced into a promoter. Therefore, no gene expression is observed in a case where the Tet repressor binds to the Tet operator sequence. By adding tetracycline or doxycycline, the Tet repressor is sequestered to allow promoter activity and turn on gene expression. The Tet operon system is widely available, such as the Tet operon used in the pcDNA (trademark) 4/TO mammalian expression vector available from Invitrogen.

In the Tet-on system, the Tet operator sequence may be located upstream or downstream of the promoter for which expression is desired to be regulated. The Tet operator sequence is preferably located downstream of a promoter capable of regulating expression in view of reducing expression leakage in a case where expression is turned off.

The specific examples of the promoter are not particularly limited, but can include a cytomegalovirus-derived promoter (CMV promoter) (including an enhancer as desired), an SV40 early promoter, a human elongation factor-1α (EF-1α) promoter, a human ubiquitin C promoter, a retrovirus Rous sarcoma virus (RSV) LTR promoter, a dihydrofolate reductase promoter, a β-actin promoter, and a phosphoglycerate kinase (PGK) promoter.

The exogenous recombinant nucleic acid means a recombinant nucleic acid introduced from the outside of a cell into the inside of the cell. The exogenous recombinant nucleic acid can be introduced into cells by methods such as transfection, transduction, and lipofection in a form of a vector containing the exogenous recombinant nucleic acid.

Transfection means that a nucleic acid is introduced into a cell across the membrane of a eukaryotic cell by chemical means (for example, using a reagent such as calcium phosphate or polyethyleneimine), mechanical means (for example, electroporation), or physical means (for example, delivery by bioballistics).

Transduction refers to introduce of a nucleic acid into a cell across a membrane of a eukaryotic cell via a virus-derived vector.

Lipofection refers to introduce of a nucleic acid into a cell by the formation of a complex between a vector and a positively charged lipid or the like by electrical interaction, and endocytosis or membrane fusion.

Preferably, in the animal cell, the exogenous recombinant nucleic acid is expressed in a constitutive expression system. The constitutive expression system is a method of expressing for 1 month or more. It is possible to maintain the expression of the target gene for a long period of time by inserting a plasmid into a chromosome, introducing an episomal vector, or introducing an exogenous nucleic acid using an artificial chromosome. In this case, in a case where the drug resistance gene is introduced into the plasmid, selection with a drug can be performed.

The recombinant nucleic acid is not particularly limited as long as it is a sequence capable of expressing a virus. Preferably, the recombinant nucleic acid is a sequence that expresses an adeno-associated virus. As described above, a sequence derived from a helper virus and a sequence derived from an adeno-associated virus is known as the sequence that expresses the adeno-associated virus, but the sequence is not limited to these as long as the adeno-associated virus can be expressed. The helper virus-derived component includes E1A, E1B, E2A, E4, and/or VA-RNA, but is not limited thereto. As the sequence derived from the adeno-associated virus, Rep and Cap are known, but the present invention is not limited thereto.

In the present invention, it is preferable that at least one of the exogenous recombinant nucleic acids contain a promoter controlled by a RNA polymerase III.

In the present invention, it is preferable that the exogenous recombinant nucleic acid is a nucleic acid encoding E2A, E4, VA-RNA, Rep, and Cap.

It is preferable that at least one of the exogenous recombinant nucleic acids contains a nucleic acid sequence that induces expression in a condition-specific manner. The promoter induced by a nucleic acid sequence that induces expression in a condition-specific manner is not particularly limited, but is preferably a doxycycline-inducible TET promoter.

In the present invention, it is preferable to use a producer cell or a packaging cell in which a Rep gene, a Cap gene, and a helper gene are incorporated into a chromosome, it is more preferable to use a producer cell in which a Rep gene, a Cap gene, and a helper gene are incorporated into a chromosome, it is still more preferable to use a producer cell in which a Rep gene, a Cap gene, a helper gene, and a GOI are incorporated into a chromosome, and it is particularly preferable to use a producer cell in which a Rep gene, a Cap gene, a helper gene, and a therapeutic or prophylactic gene are incorporated into a chromosome.

The cell in the present invention is preferably an animal cell. The animal cell is not particularly limited, but is preferably a mammalian cell or an insect cell, and more preferably a mammalian cell. Examples of the mammalian cell include a human cell, a mouse cell, a rat cell, a monkey cell, and a hamster cell, but the cells are not particularly limited. The human cell can be preferably used. Examples of the cell include mouse myeloma (NSO) cell lines, Chinese hamster ovary (CHO) cell lines, HT1080, H9, HepG2, MCF7, MDBK, Jurkat, NIH3T3, PC12, a baby hamster kidney (BHK) cell, VERO, SP2/0, YB2/0, Y0, C127, an L cell, COS (for example, COS1 and COS7), QC1-3, a human embryo-derived kidney (HEK293) cell, VERO, PER. C6, HeLa, EB1, EB2, EB3, and oncolytic or hybridoma cell lines. The cell is preferably a HEK293 cell or a CHO cell, and more preferably a HEK293 cell.

The cell in the present invention is preferably a cell adapted to growth in suspension culture. Preferably, the cell is a HEK293 cell or a CHO cell adapted to growth in suspension culture, and more preferably a HEK293 cell adapted to growth in suspension culture.

The method of the present invention includes culturing a producer cell or a packaging cell in a culture medium. The culture of cells can be performed under normal conditions for cell culture.

The cell density in the manufacturing step of the viral vector is 1 × 10⁶ cells/mL or more, more preferably 10 × 10⁶ cells/mL or more, still more preferably 20 × 10⁶ cells/mL or more, even more preferably 30 × 10⁶ cells/mL or more, even still more preferably 50 × 10⁶ cells/mL or more, even further still more preferably 70 × 10⁶ cells/mL or more, and may be 100 × 10⁶ cells/mL or more. The upper limit of the cell density is not particularly limited, but is generally 1,000 × 10⁶ cells/mL or less.

In the present invention, a proliferation step of proliferating the cell may be carried out before the manufacturing (production) step of the viral vector.

For example, before the manufacturing step, a proliferation step of culturing the cell in a culture medium not containing doxycycline and a histone deacetylase inhibitor until the cell density reaches 1 × 10⁴ cells/mL or more (more preferably 1 × 10⁵ cells/mL or more, still more preferably 1 × 10⁶ cells/mL or more, even more preferably 10 × 10⁶ cells/mL or more, even still more preferably 30 × 10⁶ cells/mL or more, and particularly preferably 100 × 10⁶ cells/mL or more) may be carried out, and then the virus manufacturing step may be carried out using a culture medium containing doxycycline and a histone deacetylase inhibitor.

The form of the culture in the proliferation step is not particularly limited, but may be carried out by perfusion culture. After the cell is proliferated by perfusion culture, a part of the cells may be transferred to a different container, and the manufacturing step may be carried out.

The culture temperature of the cells is not particularly limited, and the culture of the cells is performed at a temperature at which the cells can survive. The culture temperature is generally 25°C to 45°C, preferably 30°C to 42°C, more preferably 35°C to 40°C, and 37°C as an example.

The CO₂ concentration is generally 3 to 10% CO₂, preferably 5 to 10% CO₂, and 8% CO₂ as an example.

As the culture, batch culture, fed-batch culture, perfusion culture, or shaking culture can be carried out. It is preferable that at least a part of the virus production step is perfusion culture.

As the culture, it is preferable that the cell is in a floating state, and suspension culture is preferable.

As the culture vessel, a flask or a bioreactor can be used, but the culture container is not particularly limited.

The culture scale is not particularly limited, and the culture can be carried out in a culture medium having any volume, and for example, the cell can be cultured in a culture medium of 1 mL to 5,000 L. The lower limit is preferably 1 L, more preferably 10 L, still more preferably 50 L, and particularly preferably 100 L. The upper limit is preferably 5,000 L, more preferably 2,500 L, still more preferably 1,000 L, and particularly preferably 500 L.

The culture may be carried out while stirring by shaking. The stirring speed in the case of stirring by shaking is generally 50 rpm to 200 rpm, and preferably 80 to 150 rpm. The stirring culture may be rotary stirring culture using a propeller or the like in a reactor. The stirring speed in the case of stirring by rotating is generally 50 rpm to 200 rpm, and preferably 80 to 150 rpm. In addition, stirring may be performed by wave-type shaking stirring or vertical movement of the stirring blade, but the stirring is not particularly limited.

Examples of the culture medium include, but are not limited to, Expi293 Expression Medium (Thermo Fisher Scientific, A1435101), Dulbecco's modified Eagle's medium (DMEM) containing 10% (vol/vol) fetal bovine serum (FBS), serum-free UltraCulture (trademark) medium (Lonza), STEMPRO-34 SFM medium, human endothelial-SFM, GIBCO (registered trademark) FREESTYLE (trademark) 293 expression medium, CD CHOAGT medium, CHO-S-SFM medium, GIBCO (registered trademark) FREESTYLE (trademark) CHO expression medium, CD OPTICHO (trademark) medium, CD CHO medium, CD DG44 medium, SF-900 (trademark) medium, Expi293 (trademark) expression medium, 293 SFM medium, BalanCD (registered trademark) HEK293 medium, and any derivative or modification thereof. In a certain specific non-limiting embodiment, the high-density culture medium may be CD FORTICHO (trademark) medium, CD CHOAGT medium, CHO-S-SFM medium, GIBCO (registered trademark) FREESTYLE (trademark) CHO expression medium, CD OPTICHO (trademark) medium, CD CHO medium, CD DG44 medium, GIBCO (registered trademark) FREESTYLE (trademark) 293 expression medium, Expi293 (trademark) expression medium, LV-MAX (trademark) production medium, FREESTYLE (trademark) F17 expression medium, DYNAMIS (trademark) medium, BalanCD (registered trademark) HEK293 medium, or the same culture medium or a modification thereof.

The method for producing a viral vector according to the embodiment of the present invention may include recovering a virus. The viral vector produced in the cell that produces the viral vector remains in the cell or is released into the culture supernatant.

In a case of recovering the viral vector from the cell, for example, a sample containing the viral vector can be prepared by subjecting the cell to ultrasonic disruption, or by freezing and thawing the cell to disrupt the cell, or by bringing the cell into contact with an acidic solution or a surfactant. The specimen prepared in this way may be used as it is, or may be purified as desired.

In a case of recovering the viral vector from the culture supernatant, the culture supernatant may be used as it is as a sample containing the virus vector, or may be purified as desired.

The purification method in a case of purifying the viral vector is not particularly limited, and examples thereof include a purification method such as a CsCl gradient ultracentrifugation method, a chromatography method, or an ultrafiltration method. The viral vector can be purified from the sample containing the viral vector by the above-described purification method. Furthermore, optionally, genome DNA (gDNA) or the residual plasmid may be digested by adding MgCl₂ and Benzonase to the specimen purified as described above to perform a reaction.

The titer of the viral vector produced by the method according to the embodiment of the present invention can be measured by a conventional method known to those skilled in the art. For example, the cell culture solution after the culture is collected, the cells are disrupted by freeze-thaw, and then the supernatant is collected by centrifugation. The gDNA (genomic DNA) and the residual plasmid can be digested by adding MgCl₂ and Benzonase to the collected supernatant to react. The titer of the viral vector can be measured by using the sample containing the viral vector obtained as described above as a droplet digital PCR (ddPCR) sample and carrying out ddPCR.

Furthermore, the Full ratio can be calculated from the ratio of the capsid particle titer (Vp/mL) measured using the ELISA kit to the genome titer (vg/mL) calculated by ddPCR measurement. Alternatively, the Full/Empty ratio may be obtained by analyzing the sample solution after virus extraction and purification by HPLC measurement, capillary isoelectric focusing, or the like in the same measurement system.

In an example of the present invention, by carrying out the culture in the presence of a histone deacetylase inhibitor, the production amount of the viral vector such as AAV can be preferably increased by 1.5 times or more, more preferably 2 times or more, and still more preferably 3 times or more, as compared with a case where the histone deacetylase inhibitor is not used.

In an example of the present invention, in the virus produced in the culture step, the ratio (Full rate) of the amount of the capsid containing the full-length gene to the total amount of the capsid is preferably 5% or more, more preferably 10% or more, still more preferably 20% or more, even still more preferably 25% or more, and particularly preferably 30% or more.

The present invention will be more specifically described based on the following examples, but the present invention is not limited to the examples.

### Examples

### [Materials and methods]

### <Cell maintenance culture>

A plasmid (SEQ ID NO: 1 and SEQ ID NO: 2) was introduced into HEK293 cells (Thermo Fisher Scientific, A49784) adapted to growth in suspension culture, and a stable expression cell line (AAV producer cell) in which the sequence 1 (including a reverse tetracycline-controlled transactivator (rtTA) gene, a Rep gene, a Cap gene, an E2 gene, an E4 gene, and a GFP gene (as a GOI) sandwiched between ITR sequences, in which the Rep gene, the Cap gene, the E2 gene, and the E4 gene are under the control of a promoter having a tetracycline response element (TRE) sequence) was incorporated into the chromosome of the cell was cultured in a BalanCD HEK293 medium (FUJIFILM Irvine Scientific, 551-34231). The cells were suspended so that the cell density was 2 × 10⁶ to 30 × 10⁶ cells/mL, and maintained in a 125 mL shaking flask. The culture solution was incubated at 37°C and 8% CO₂ while being stirred at a constant rate of 120 rpm.

### <Perfusion culture>

280 mL of a BalanCD HEK293 medium (FUJIFILM Irvine Scientific, 551-34231) was seeded with AAV producer cells suspended at 0.5 × 10⁶ cells/mL in a 0.5 L reactor (Biott Corporation). The perfusion culture was performed using a BalanCD HEK293 medium (FUJIFILM Irvine Scientific, 551-34231) and a BalanCD HEK293 Feed (FUJIFILM Irvine Scientific, 550-34245) as a perfusion medium.

### <Plasmid construction>

The entire sequences of the plasmids used are shown in SEQ ID NOs: 1 to 5 in the sequence listing. The sequence was manufactured by a total synthesis method.

**[Table 1]**

| SEQ ID NO: | Sequence name: description |
|---|---|
| SEQ ID NO: 1 | Plasmid encoding AAV vector production gene |
| SEQ ID NO: 2 | Plasmid encoding transposase |
| SEQ ID NO: 3 | ddPCR-Primer F: primer for detecting AAV genome |
| SEQ ID NO: 4 | ddPCR-Primer R: primer for detecting AAV genome |
| SEQ ID NO: 5 | Probe for detecting AAV genome, modified with FAM at 5' end and BHQ1 at 3' end |

### <Measurement of AAV titer>

The HEK cell culture solution, which had been cultured for 72 hours after the gene transfer under the conditions of 37°C and 8% CO₂, was collected, and the cells were disrupted by freezing and thawing at -80°C. Then, the supernatant was collected by centrifugation at 13,800 ×g, 2 mmol/L (final concentration) MgCl₂, and 25 U/mL Benzonase was added thereto, and a reaction was carried out at 37°C for 2 hours to digest gDNA (genomic DNA) and residual plasmid. The sample after the reaction was incubated at 95°C for 15 minutes, at 70°C for 3 minutes, at 40°C for 3 minutes, and at 20°C for 3 minutes in this order to inactivate Benzonase, and the sample was used as a ddPCR (Droplet Digital PCR) sample. The ddPCR sample was diluted 50 times with a TE buffer (pH 8.0, containing 0.05% Pluronic F-68 and 10 µg/mL of bovine thymus DNA). 10 µL of ddPCR(trademark)Supermix for Probes (no dUTP) (Bio-Rad Laboratories, Inc.), 2 µL of the diluted ddPCR sample, a primer (SEQ ID NO: 3, SEQ ID NO: 4, final concentration: 900 nmol/L), a probe (SEQ ID NO: 5, final concentration: 250 nmol/L), and nuclease-free water (Thermo Fisher Scientific, 10977015) were mixed in a tube on ice, and the total liquid volume was adjusted to 22 µL to perform ddPCR. In the ddPCR, droplets were formed from the preparation liquid by a droplet generator, and were incubated in the order of 95°C for 10 minutes (preheating), the denaturation, annealing, and extension of 94°C for 30 seconds (denaturation) and then 55°C for 60 seconds (annealing and extension) for 40 cycles, and 98°C for 10 minutes. Measurement was performed using a droplet reader, and the AAV genomic titer (vg/mL) was calculated.

### Experiment 1:

### [Method]

AAV producer cells were seeded in a 24-well plate at 1 × 10⁶ cells/mL, and doxycycline (final concentration: 0.5 µg/mL) and Epigenetics Screening Library (CAYMAN, 11076) were added at final concentrations of 2.5 µmol/L and 5.0 µmol/L. After the treatment for 72 hours, the cells were recovered, and the AAV titer was measured.

### <Results>

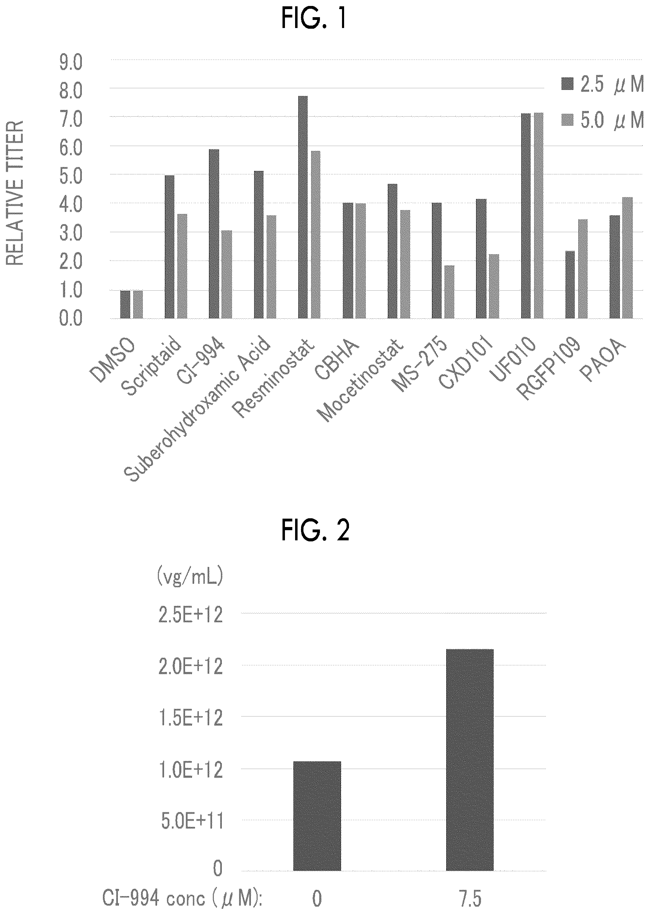

A compound that improves the titer with respect to the titer at the time of DMSO addition was selected, the results of the titer measurement are shown in FIG. 1, and the list of compounds is shown in Table 2. As a result of the screening, it was found that the HDAC inhibitor improves the titer.

**[Table 2]**

| Table 2: List of compounds with improved titer | |
|---|---|
| No. | Compound name |
| 1 | Scriptaid |
| 2 | CI-994 |
| 3 | Suberohydroxamic Acid |
| 4 | Resminostat |
| 5 | CBHA |
| 6 | Mocetinostat |
| 7 | MS-275 |
| 8 | CXD 101 |
| 9 | UF010 |
| 10 | RGFP109 |
| 11 | PAOA |

### Experiment 2:

### [Method]

The AAV producer cells were cultured at 30 × 10⁶ cells/mL by perfusion culture in a reactor, and then 24 mL of the culture solution was transferred to a 125 mL flask. Doxycycline (final concentration: 5 µg/mL) and CI-994 (CAYMAN, 11076) were added to the cell culture solution at a final concentration of 7.5 µmol/L. 140 µL of BalanCD HEK293 Feed (FUJIFILM Irvine Scientific, 550-34245) and 10 µL of a 45 w/v% D(+) -glucose solution (FUJIFILM Wako Pure Chemical Corporation, 079-05511) were added to 1 mL of the cell culture solution every other day to maintain the cells. After the drug addition, the cells were recovered after the treatment for 72 hours, and the AAV titer was measured.

### <Results>

The results of the titer measurement are shown in FIG. 2. It was found that the titer of the sample treated with CI-994 (CI-994 conc: 7.5 µM) was 2.2 × 10¹² vg/mL with respect to the titer of 1.1 × 10¹² vg/mL of the sample (CI-994 conc: 0 µM) not treated with CI-994, and thus the titer improvement effect was observed for the cell culture solution with a high density.

The same titer improvement effect was also observed for the HDAC inhibitors shown in Table 2 other than CI-994.

### Experiment 3:

### [Method]

As in Experiment 2, the AAV producer cells were cultured at 30 × 10⁶ cells/mL or more by perfusion culture in a reactor, and the cells were collected into a flask to examine the production of AAV. 30% of the total amount of the cell culture solution was collected from the reactor every 24 hours and diluted with a fresh culture medium to maintain a cell density of 30 × 10⁶ cells/mL or more for 7 days, 24 mL of the collected cell culture solution was transferred to a 125 mL flask, and doxycycline (final concentration: 5 µg/mL) and CI-994 (CAYMAN, 11076) were added to the cell culture solution at a final concentration of 7.5 µmol/L. 140 µL of BalanCD HEK293 Feed (FUJIFILM Irvine Scientific, 550-34245) and 10 µL of a 45 w/v% D(+) -glucose solution (FUJIFILM Wako Pure Chemical Corporation, 079-05511) were added to 1 mL of the cell culture solution every other day to maintain the cells. After the drug addition, the cells were recovered after the treatment for 72 hours, and the AAV titer was measured.

### <Results>

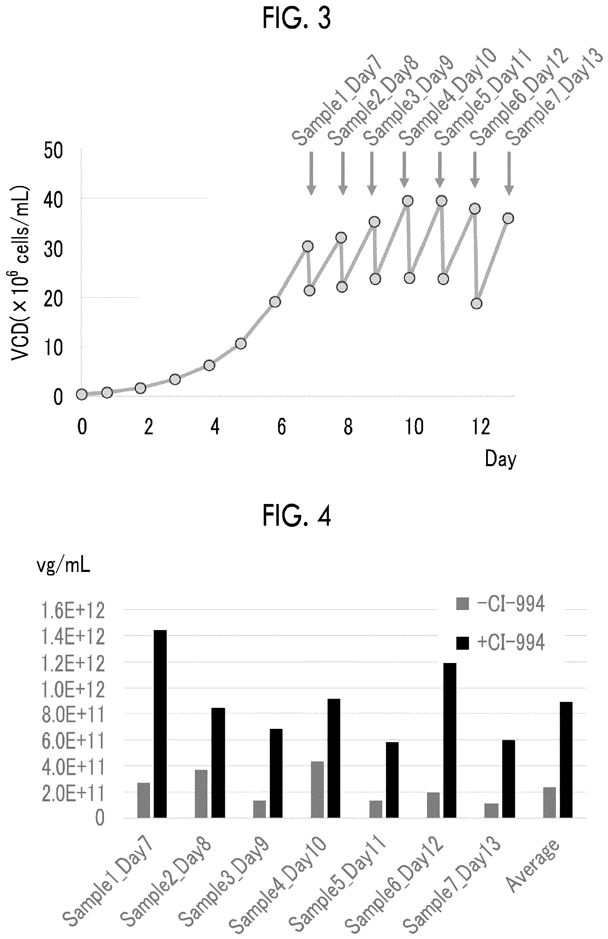

FIG. 3 shows the change in the cell density in the reactor, and FIG. 4 shows the titer measurement results of AAV produced in the cell culture solution collected every 24 hours. It was confirmed that the CI-994-treated sample (+CI-994) exhibited a higher titer than the CI-994-untreated sample (-CI-994) under the conditions in which the AAV was continuously produced in another culture vessel every 24 hours while maintaining a high density of the cell density in the reactor, and thus the titer improvement effect by the HDAC inhibitor was observed.

### Experiment 4:

### [Method]

The AAV was extracted and purified from the sample on Day 7 in Experiment 3 using an AAVpro Purification Kit (Takara Bio Inc., 6675). For the sample solution after the purification, the area value of each of the peaks of the Empty and Full particles of AAV was measured using high performance liquid chromatography, and the Full rate (the ratio of the amount of capsids containing the full-length gene to the total amount of capsids) was calculated. An anion exchange column was used as a separation column, and a fluorescence detector (excitation: 280 nm, detection: 348 nm) was used for detection.

### <Results>

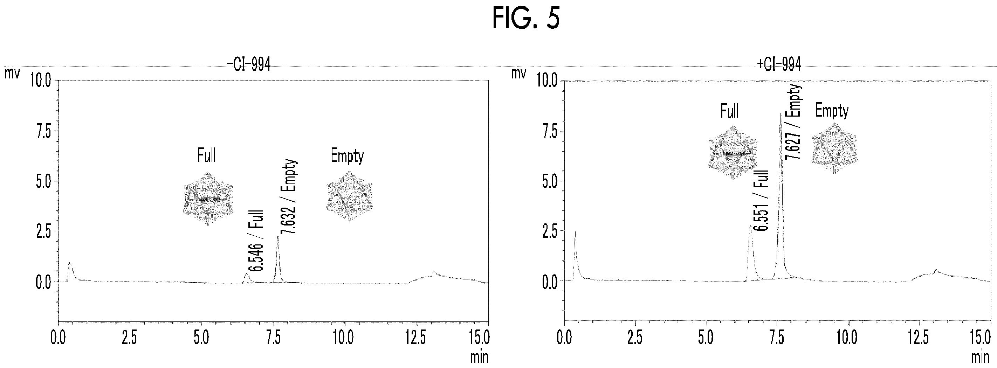

FIG. 5 shows the measurement results of the CI-994-untreated sample (-CI-994) and the CI-994-treated sample (+CI-994) obtained using high performance liquid chromatography. The peak area was measured, and a list in which the area ratio of the peak indicating the Full particle to the peak indicating the Empty particle was calculated is shown in Table 3. It was found that the CI-994 treatment had an improvement effect on the Full rate.

**[Table 3]**

| Table 3: Measurement value of Full rate | | | |
|---|---|---|---|
| | Area | | Full rate (%) |
| Sample | RT: 6.5 min | RT: 7.6 min | |
| | Full | Empty | |
| Without CI-994 | 5700 | 18275 | 23.8 |
| With CI-994 | 33878 | 75986 | 30.8 |

### Experiment 5:

### [Method]

The AAV producer cell was seeded in a 125 mL flask at 0.2 × 10⁶ cells/mL, 1.0 × 10⁶ cells/mL, 6.0 × 10⁶ cells/mL, and 24 × 10⁶ cells/mL, doxycycline (final concentration: 0.5 µg/mL) was added thereto, and after 72 hours of treatment, the cells were recovered and the AAV titer was measured.

### <Results>

The titer of the AAV producer cell decreases as the cell density increases. Specifically, in a case where the cell density is 0.2 × 10⁶ cells/mL to 1.0 × 10⁶ cells/mL, the titer is about 1/5. In a case where the HDAC inhibitor is added at a cell density of 1.0 × 10⁶ cells/mL or more to produce the virus, the decrease in titer is suppressed as compared with a case where the HDAC inhibitor is not added, or a titer equal to or higher than the titer of 0.2 × 10⁶ cells/mL (without the HDAC inhibitor) is obtained.

### Experiment 6:

### [Method]

The AAV producer cell was seeded in a 125 mL flask at 0.2 × 10⁶ cells/mL, 1.0 × 10⁶ cells/mL, 6.0 × 10⁶ cells/mL, 24 × 10⁶ cells/mL, and 30 × 10⁶ cells/mL, doxycycline (final concentration: 0.5 µg/mL) was added thereto alone, and after 72 hours of treatment, the cells were recovered and the AAV titer was measured.

The AAV producer cell was seeded in a 125 mL flask at 0.2 × 10⁶ cells/mL, 1.0 × 10⁶ cells/mL, 8.0 × 10⁶ cells/mL, and 30 × 10⁶ cells/mL, doxycycline (final concentration: 0.5 µg/mL) and CI-994 (final concentration: 7.5 µmol/mL) were added thereto, and after 72 hours of treatment, the cells were recovered and the AAV titer was measured.

### <Results>

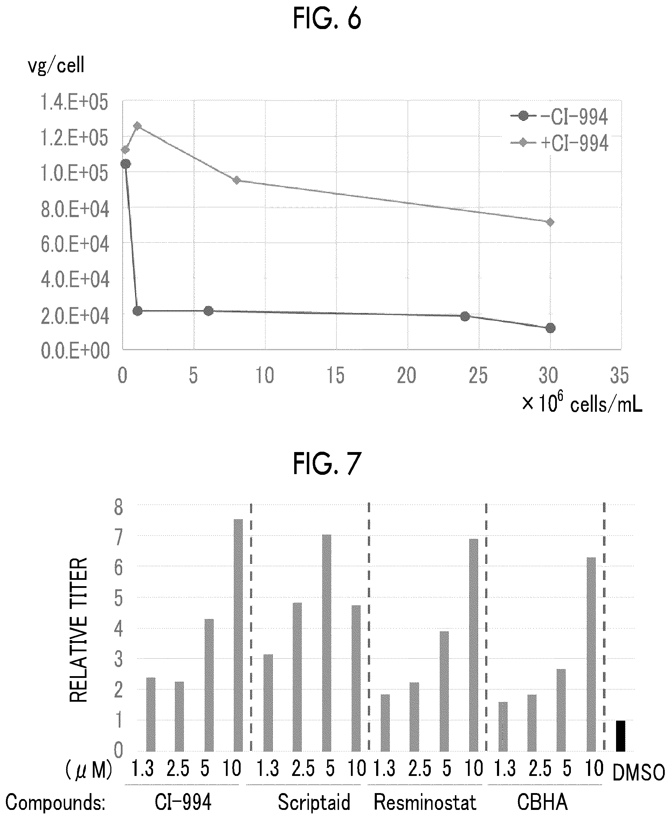

In the AAV producer cells, as the cell density increased, the titer per cell decreased (FIG. 6). Specifically, in a case where the cell density was 0.2 × 10⁶ cells/mL to 1.0 × 10⁶ cells/mL, the titer was about 1/5, but in a case where CI-994 was added at a cell density of 1.0 × 10⁶ cells/mL or more to produce the virus, the decrease in titer was suppressed, and a titer equal to the titer of 0.2 × 10⁶ cells/mL (without the CI-994 treatment) was obtained.

### Experiment 7:

### [Method]

The AAV producer cell was seeded in a 24-well plate at 1 × 10⁶ cells/mL, and doxycycline (final concentration: 0.5 µg/mL) and various HDAC inhibitors (CI-994, Scriptaid, Resminostat, and CBHA) were added thereto at final concentrations of 1.3 µmol/L, 2.5 µmol/L, 5 µmol/L, and 10 µmol/L. After 96 hours of treatment, the cells were recovered and the AAV titer was measured.

### <Results>

Compounds that improve the titer with respect to the titer at the time of DMSO addition were selected, and the results of the titer measurement are shown in FIG. 7. It was confirmed that the various compounds have a particularly high effect of improving the titer at a concentration of 5 µmol/L or more.

### [Consideration]

In the present technology, it is speculated that in a strain (AAV producer cell) in which a gene necessary for AAV vector expression is inserted into a chromosome of a host cell, the activity of histone deacetylase (HDAC) expressed in the cell is inhibited, thereby enhancing histone acetylation and inducing a change in chromatin structure, which contributes to the improvement of the AAV titer and the full rate. In general, it is known that histone acetylation causes decondensation of the chromatin structure, and it is considered that the expression of the gene necessary for AAV production, which is inserted, is improved, and the titer is improved. In addition, it is speculated that the decondensation of the chromatin structure also contributes to the improvement of intracellular replication of the GOI encapsulated in the AAV vector, which leads to the improvement of the full particles.
SEQ ID NO: 1: 
ATGACTCTCTTAAGGTAGCCAAATTTCCATAGGCTCCGCCCCCCTGACGAG
CATCACAAAAATCGACGCTCAAGTCAGAGGTGGCGAAACCCGACAGGACTATAAA
GATACCAGGCGTTTCCCCCTGGAAGCTCCCTCGTGCGCTCTCCTGTTCCGACCCTG
 
CCGCTTACCGGATACCTGTCCGCCTTTCTCCCTTCGGGAAGCGTGGCGCTTTCTCAT
AGCTCACGCTGTAGGTATCTCAGTTCGGTGTAGGTCGTTCGCTCCAAGCTGGGCTG
TGTGCACGAACCCCCCGTTCAGCCCGACCGCTGCGCCTTATCCGGTAACTATCGTCT
TGAGTCCAACCCGGTAAGACACGACTTATCGCCACTGGCAGCAGCCACTGGTAAC
AGGATTAGCAGAGCGAGGTATGTAGGCGGTGCTACAGAGTTCTTGAAGTGGTGGCC
TAACTACGGCTACACTAGAAGGACAGTATTTGGTATCTGCGCTCTGCTGAAGCCAG
TTACCTTCGGAAAAAGAGTTGGTAGCTCTTGATCCGGCAAACAAACCACCGCTGGT
AGCGGTGGTTTTTTTGTTTGCAAGCAGCAGATTACGCGCAGAAAAAAAGGATCTCA
AGAAGATCCTTTGATCTTTTCTACGGGGTCTGACGCTCAGTGGAACGAAAACTCAC
GTTAAGGGATTTTGGTCATGAGATTATCAAAAAGGATCTTCACCTAGATCCTTTTAA
ATTAAAAATGAAGTTTTAAATCAATCTAAAGTATATATGAGTAAACTTGGTCTGACA
Gttagaaaaactcatcgagcatcaaatgaaactgcaatttattcatatcaggattatcaataccatatttttgaaaaagccgtttctgtaatga
aggagaaaactcaccgaggcagttccataggatggcaagatcctggtatcggtctgcgattccgactcgtccaacatcaatacaacctat
taatttcccctcgtcaaaaataaggttatcaagtgagaaatcaccatgagtgacgactgaatccggtgagaatggcaaaagtttatgcattt
ctttccagacttgttcaacaggccagccattacgctcgtcatcaaaatcactcgcatcaaccaaaccgttattcattcgtgattgcgcctgag
cgaaacgaaatacgcgatcgctgttaaaaggacaattacaaacaggaatcgaatgcaaccggcgcaggaacactgccagcgcatcaa
caatattttcacctgaatcaggatattcttctaatacctggaatgctgttttcccagggatcgcagtggtgagtaaccatgcatcatcaggagt
acggataaaatgcttgatggtcggaagaggcataaattccgtcagccagtttagtctgaccatctcatctgtaacatcattggcaacgctac
ctttgccatgtttcagaaacaactctggcgcatcgggcttcccatacaatcgatagattgtcgcacctgattgcccgacattatcgcgagcc
catttatacccatataaatcagcatccatgttggaatttaatcgcggcctagagcaagacgtttcccgttgaatatggctcatactcttcctttt
caatattattgaagcatttatcagggttattgtctcatgagcggatacatatttgaatgtatttagaaaaataaacaaataggggttccgcgC
ACATTTCCCCGAAAAGTGCCACCTCCCATTCGCCATTCAGGCTGCGCAACTGTTGG
GAAGGGCGATCGGTGCGGGCCTCTTCGCTATTACGCCAGCTGGCGAAAGGGGGAT
GTGCTGCAAGGCGATTAAGTTGGGTAACGCCAGGGTTTTCCCAGTCACGACGTTGT
AAAACGACGGCCAGTGAGCGCGCCTCGTTCATTCACGTTTTTGAACCCGTGGAGG
ACGGGCAGACTCGCGGTGCAAATGTGTTTTACAGCGTGATGGAGCAGATGAAGAT
GCTCGACACGCTGCAGAACACGCAGCTAGATTAACCCTAGAAAGATAATCATATTG
TGACGTACGTTAAAGATAATCATGCGTAAAATTGACGCATGTGTTTTATCGGTCTGTA
TATCGAGGTTTATTTATTAATTTGAATAGATATTAAGTTTTATTATATTTACACTTACAT
ACTAATAATAAATTCAACAAACAATTTATTTATGTTTATTTATTTATTAAAAAAAAAC
AAAAACTCAAAATTTCTTCTATAAAGTAACAAAACTTTTATCGAATTCCTGCAGCCC
GGGGGGACAGCCCCCCCCCAAAGCCCCCAGGGATGTAATTACGTCCCTCCCCCGC
TAGGGGGCAGCAGCGAGCCGCCCGGGGCTCCGCTCCGGTCCGGCGCTCCCCCCGC
 
ATCCCCGAGCCGGCAGCGTGCGGGGACAGCCCGGGCACGGGGAAGGTGGCACGG
GATCGCTTTCCTCTGAACGCTTCTCGCTGCTCTTTGAGCCTGCAGACACCTGGGGG
GATACGGGGAAAAGGCCTCCAAGGCCAGCTTCCCACAATAAGTTGGGTGAATTTTG
GCTCATTCCTCCTTTCTATAGGATTGAGGTCAGAGCTTTGTGATGGGAATTCTGTGG
AATGTGTGTCAGTTAGGGTGTGGAAAGTCCCtttactccctatcagtgatagagaacgtatgaagagtttact
ccctatcagtgatagagaacgtatgcagactttactccctatcagtgatagagaacgtataaggagtttactccctatcagtgatagagaac
gtatgaccagtttactccctatcagtgatagagaacgtatctacagtttactccctatcagtgatagagaacgtatatccagtttactccctatc
agtgatagagaacgtatgtcgaggtaggcgtgtacggtgggcgcctataaaagcagagctcgtttagtgaaccgtcagatcgcctgga
gcaattccacaacacttttgtcttatacttggtttagtgaaccgtcagatcagatctttgtcgatcctaccatccactcgacacacccgccagc
ggccgTTTAAAcagttgcgcagccatcgacgtcagacgcggaagcttcgatcaactacgcagacaggtaccaaaacaaatgttct
cgtcacgtgggcatgaatctgatgctgtttccctgcagacaatgcgagagaatgaatcagaattcaaatatctgcttcactcacggacaga
aagactgtttagagtgctttcccgtgtcagaatctcaacccgtttctgtcgtcaaaaaggcgtatcagaaactgtgctacattcatcatatcat
gggaaaggtgccagacgcttgcactgcctgcgatctggtcaatgtggatttggatgactgcatctttgaacaataaatgatttaaatcaggt
atgtcttttgttgatcaccctccagattggttggaagaagttggtgaaggtcttcgcgagtttttgggccttgaagcgggcccaccgaaacc
aaaacccaatcagcagcatcaagatcaagcccgtggtcttgtgctgcctggttataactatctcggacccggaaacggtctcgatcgagg
agagcctgtcaacagggcagacgaggtcgcgcgagagcacgacatctcgtacaacgagcagcttgaggcgggagacaacccctac
ctcaagtacaaccacgcggacgccgagtttcaggagaagctcgccgacgacacatccttcgggggaaacctcggaaaggcagtcttt
caggccaagaaaagggttctcgaaccttttggcctggttgaagagggtgctaagacggcccctaccggaaagcggatagacgaccac
tttccaaaaagaaagaaggctcggaccgaagaggactccaagccttccacctcgtcagacgccgaagctggacccagcggatcccag
cagctgcaaatcccagcccaaccagcctcaagtttgggagctgatacaatgtctgcgggaggtggcggcccattgggcgacaataacc
aaggtgccgatggagtgggcaatgcctcgggagattggcattgcgattccacgtggatgggggacagagtcgtcaccaagtccaccc
gaacctgggtgctgcccagctacaacaaccaccagtaccgagagatcaaaagcggctccgtcgacggaagcaacgccaacgcctac
tttggatacagcaccccctgggggtactttgactttaaccgcttccacagccactggagcccccgagactggcaaagactcatcaacaac
tactggggcttcagaccccggtccctcagagtcaaaatcttcaacattcaagtcaaagaggtcacggtgcaggactccaccaccaccat
cgccaacaacctcacctccaccgtccaagtgtttacggacgacgactaccagctgccctacgtcgtcggcaacgggaccgagggatg
cctgccggccttccctccgcaggtctttacgctgccgcagtacggttacgcgacgctgaaccgcgacaacacagaaaatcccaccgag
aggagcagcttcttctgcctagagtactttcccagcaagatgctgagaacgggcaacaactttgagtttacctacaactttgaggaggtgc
ccttccactccagcttcgctcccagtcagaacctgttcaagctggccaacccgctggtggaccagtacttgtaccgcttcgtgagcacaa
ataacactggcggagtccagttcaacaagaacctggccgggagatacgccaacacctacaaaaactggttcccggggcccatgggcc
gaacccagggctggaacctgggctccggggtcaaccgcgccagtgtcagcgccttcgccacgaccaataggatggagctcgagggc
gcgagttaccaggtgcccccgcagccgaacggcatgaccaacaacctccagggcagcaacacctatgccctggagaacactatgatc
ttcaacagccagccggcgaacccgggcaccaccgccacgtacctcgagggcaacatgctcatcaccagcgagagcgagacgcagc
cggtgaaccgcgtggcgtacaacgtcggcgggcagatggccaccaacaaccagagctccaccactgcccccgcgaccggcacgta
 
caacctccaggaaatcgtgcccggcagcgtgtggatggagagggacgtgtacctccaaggacccatctgggccaagatcccagaga
cgggggcgcactttcacccctctccggccatgggcggattcggactcaaacacccaccgcccatgatgctcatcaagaacacgcctgt
gcccggaaatatcaccagcttctcggacgtgcccgtcagcagcttcatcacccagtacagcaccgggcaggtcaccgtggagatgga
gtgggagctcaagaaggaaaactccaagaggtggaacccagagatccagtacacaaacaactacaacgacccccagtttgtggacttt
gccccggacagcaccggggaatacagaaccaccagacctatcggaacccgataccttacccgacccctttaaGTTTAAAcccg
ggagttctagggatctgcccctctccctcccccccccctaacgttactggccgaagccgcttggaataaggccggtgtgcgtttgtctata
tgttattttccaccatattgccgtcttttggcaatgtgagggcccggaaacctggccctgtcttcttgacgagcattcctaggggtctttcccc
tctcgccaaaggaatgcaaggtctgttgaatgtcgtgaaggaagcagttcctctggaagcttcttgaagacaaacaacgtctgtagcgac
cctttgcaggcagcggaaccccccacctggcgacaggtgcctctgcggccaaaagccacgtgtataagatacacctgcaaaggcggc
acaaccccagtgccacgttgtgagttggatagttgtggaaagagtcaaatggctctcctcaagcgtattcaacaaggggctgaaggatg
cccagaaggtaccccattgtatgggatctgatctggggcctcggtgcacatgctttacatgtgtttagtcgaggttaaaaaaacgtctagg
ccccccgaaccacggggacgtggttttcctttgaaaaacacgatgataaggatccaccggaggccaccatggccagtcgggaagagg
agcagcgcgaaaccacccccgagcgcggacgcggtgcggcgcgacgtcccccaaccatggaggacgtgtcgtccccgtccccgtc
gccgccgcctccccgggcgcccccaaaaaagcggatgaggcggcgtatcgagtccgaggacgaggaagactcatcacaagacgc
gctggtgccgcgcacacccagcccgcggccatcgacctcggcggcggatttggccattgcgcccaagaagaaaaagaagcgcccctt
ctcccaagcccgagcgcccgccatcaccagaggtaatcgtggacagcgaggaagaaagagaagatgtggcgctacaaatggtgggt
ttcagcaacccaccggtgctaatcaagcatggcaaaggaggtaagcgcacagtgcggcggctgaatgaagacgacccagtggcgcg
tggtatgcggacgcaagaggaagaggaagagcccagcgaagcggaaagtgaaattacggtgatgaacccgctgagtgtgccgatcg
tgtctgcgtgggagaagggcatggaggctgcgcgcgcgctgatggacaagtaccacgtggataacgatctaaaggcgaacttcaaac
tactgcctgaccaagtggaagctctggcggccgtatgcaagacctggctgaacgaggagcaccgcgggttgcagctgaccttcacca
gcaacaagacctttgtgacgatgatggggcgattcctgcaggcgtacctgcagtcgtttgcagaggtgacctacaagcatcacgagccc
acgggctgcgcgttgtggctgcaccgctgcgctgagatcgaaggcgagcttaagtgtctacacggaagcattatgataaataaggagc
acgtgattgaaatggatgtgacgagcgaaaacgggcagcgcgcgctgaaggagcagtctagcaaggccaagatcgtgaagaaccg
gtggggccgaaatgtggtgcagatctccaacaccgacgcaaggtgctgcgtgcacgacgcggcctgtccggccaatcagttttccggc
aagtcttgcggcatgttcttctctgaaggcgcaaaggctcaggtggcttttaagcagatcaaggcttttatgcaggcgctgtatcctaacgc
ccagaccgggcacggtcaccttttgatgccactacggtgcgagtgcaactcaaagcctgggcacgcgcccctttttgggaaggcagcta
ccaaagttgactccgttcgccctgagcaacgcggaggacctggacgcggatctgatctccgacaagagcgtgctggccagcgtgcac
cacccggcgctgatagtgttccagtgctgcaaccctgtgtatcgcaactcgcgcgcgcagggcggaggccccaactgcgacttcaaga
tatcggcgcccgacctgctaaacgcgttggtgatggtgcgcagcctgtggagtgaaaacttcaccgagctgccgcggatggttgtgcct
gagtttaagtggagcactaaacaccagtatcgcaacgtgtccctgccagtggcgcatagcgatgcgcggcagaacccctttgatttttaa
cccgggagttctagggatctgTTAATTAAGCGGCCGCTCGAGTCTAGAGGGCCCGTTTAAGCCG
CTTGGAATAAGGCCGGTGTGCGTTTGTCTATATGTTATTTTccaccatattgccgtcttttggcaatgt
gagggcccggaaacctggccctgtcttcttgacgagcattcctaggggtctttcccctctcgccaaaggaatgcaaggtctgttgaatgtc
 
gtgaaggaagcagttcctctggaagcttcttgaagacaaacaacgtctgtagcgaccctttgcaggcagcggaaccccccacctggcg
acaggtgcctctgcggccaaaagccacgtgtataagatacacctgcaaaggcggcacaaccccagtgccacgttgtgagttggatagtt
gtggaaagagtcaaatggctcccctcaagcgtattcaacaaggggctgaaggatgcccagaaggtaccccattgtatgggatctgatct
ggggcctcggtgcacatgctttacatgtgtttagtcgaggttaaaaaacgtctaggccccccgaaccacggggacgtggttttcctttgaaa
aacacgatgataatacATGACTACGTCCGGCGTTCCATTTGGCATGACACTACGACCAACAC
GATCTCGGTTGTCTCGGCGCACTCCGTACAGTAGGGATCGCCTACCTCCTTTTGAGA
CAGAGACCCGCGCTACCATACTGGAGGATCATCCGCTGCTGCCCGAATGTAACACT
TTGACAATGCACAACGTGAGTTACGTGCGAGGTCTTCCCTGCAGTGTGGGATTTAC
GCTGATTCAGGAATGGGTTGTTCCCTGGGATATGGTTCTGACGCGGGAGGAGCTTG
TAATCCTGAGGAAGTGTATGCACGTGTGCCTGTGTTGTGCCAACATTGATATCATGA
CGAGCATGATGATCCATGGTTACGAGTCCTGGGCTCTCCACTGTCATTGTTCCAGTC
CCGGTTCCCTGCAGTGCATAGCCGGCGGGCAGGTTTTGGCCAGCTGGTTTAGGATG
GTGGTGGATGGCGCCATGTTTAATCAGAGGTTTATATGGTACCGGGAGGTGGTGAAT
TACAACATGCCAAAAGAGGTAATGTTTATGTCCAGCGTGTTTATGAGGGGTCGCCA
CTTAATCTACCTGCGCTTGTGGTATGATGGCCACGTGGGTTCTGTGGTCCCCGCCAT
GAGCTTTGGATACAGCGCCTTGCACTGTGGGATTTTGAACAATATTGTGGTGCTGTG
CTGCAGTTACTGTGCTGATTTAAGTGAGATCAGGGTGCGCTGCTGTGCCCGGAGGA
CAAGGCGTCTCATGCTGCGGGCGGTGCGAATCATCGCTGAGGAGACCACTGCCATG
TTGTATTCCTGCAGGACGGAGCGGCGGCGGCAGCAGTTTATTCGCGCGCTGCTGCA
GCACCACCGCCCTATCCTGATGCACGATTATGACTCTACCCCCATGTAGGCGGCCGC
TCGAGTCTAGAGGGCCCTTAATTAACCGCTGATCAGCCTCGACTGTGCCTTCTAGTT
GCCAGCCATCTGTTGTTTGCCCCTCCCCCGTGCCTTCCTTGACCCTGGAAGGTGCC
ACTCCCACTGTCCTTTCCTAATAAAATGAGGAAATTGCATCGCATTGTCTGAGTAGG
TGTCATTCTATTCTGGGGGGTGGGGTGGGGCAGGACAGCAAGGGGGAGGATTGGG
AAGACAATAGCAGGCATGCTGGGGATGCGGTGGGCTCTATGGTCATGTCTGTAAGC
GGCCGCTACGTCGACTTAAGCTTGGTACCGAGCTCGGATCCCCAGTGTGGTGGAAT
TCCTGCTTCGCGATGTACGGGCCAGATATACGCGTTGTTTACGCTCCGGTGCCCGTC
AGTGGGCAGAGCGCACATCGCCCACAGTCCCCGAGAAGTTGGGGGGAGGGGTCG
GCAATTGAACCGGTGCCTAGAGAAGGTGGCGCGGGGTAAACTGGGAAAGTGATGT
CGTGTACTGGCTCCGCCTTTTTCCCGAGGGTGGGGGAGAACCGTATATAAGTGCAG
TAGTCGCCGTGAACGTTCTTTTTCGCAACGGGTTTGCCGCCAGAACACAGGTAAGT
GCCGTGTGTGGTTCCCGCGGGCCTGGCCTCTTTACGGGTTATGGCCCTTGCGTGCCT
TGAATTACTTCCACGCCCCTGGCTGCAGTACGTGATTCTTGATCCCGAGCTTCGGGT
 
TGGAAGTGGGTGGGAGAGTTCGAGGCCTTGCGCTTAAGGAGCCCCTTCGCCTCGT
GCTTGAGTTGAGGCCTGGCTTGGGCGCTGGGGCCGCCGCGTGCGAATCTGGTGGC
ACCTTCGCGCCTGTCTCGCTGCTTTCGATAAGTCTCTAGCCATTTAAAATTTTTGATG
ACCTGCTGCGACGCTTTTTTTCTGGCAAGATAGTCTTGTAAATGCGGGCCAAGATCT
GCA
 
CACTGGTATTTCGGTTTTTGGGGCCGCGGGCGGCGACGGGGCCCGTGCGTC
CCAGCGCACATGTTCGGCGAGGCGGGGCCTGCGAGCGCGGCCACCGAGAATCGGA
CGGGGGTAGTCTCAAGCTGGCCGGCCTGCTCTGGTGCCTGGCCTCGCGCCGCCGTG
TATCGCCCCGCCCTGGGCGGCAAGGCTGGCCCGGTCGGCACCAGTTGCGTGAGCG
GAAAGATGGCCGCTTCCCGGCCCTGCTGCAGGGAGCTCAAAATGGAGGACGCGGC
GCTCGGGAGAGCGGGCGGGTGAGTCACCCACACAAAGGAAAAGGGCCTTTCCGT
CCTCAGCCGTCGCTTCATGTGACTCCACGGAGTACCGGGCGCCGTCCAGGCACCTC
GATTAGTTCTCGAGCTTTTGGAGTACGTCGTCTTTAGGTTGGGGGGAGGGGTTTTAT
GCGATGGAGTTTCCCCACACTGAGTGGGTGGAGACTGAAGTTAGGCCAGCTTGGC
ACTTGATGTAATTCTCCTTGGAATTTGCCCTTTTTGAGTTTGGATCTTGGTTCATTCT
CAAGCCTCAGACAGTGGTTCAAAGTTTTTTTCTTCCATTTCAGGTGTCGTGAACCT
GCAGGATTTAAATTCTAGttcaccatgtctagactggacaagagcaaagtcataaactctgctctggaattactcaatgga
gtcggtatcgaaggcctgacgacaaggaaactcgctcaaaagctgggagttgagcagcctaccctgtactggcacgtgaagaacaag
cgggccctgctcgatgccctgccaatcgagatgctggacaggcatcatacccactcctgccccctggaaggcgagtcatggcaagact
ttctgcggaacaacgccaagtcataccgctgtgctctcctctcacatcgcgacggggctaaagtgcatctcggcacccgcccaacagag
aaacagtacgaaaccctggaaaatcagctcgcgttcctgtgtcagcaaggcttctccctggagaacgcactgtacgctctgtccgccgtg
ggccactttacactgggctgcgtattggaggaacaggagcatcaagtagcaaaagaggaaagagagacacctaccaccgattctatgc
ccccacttctgaaacaagcaattgagctgttcgaccggcagggagccgaacctgccttccttttcggcctggaactaatcatatgtggcct
ggagaaacagctaaagtgcgaaagcggcgggccgaccgacgcccttgacgattttgacttagacatgctcccagccgatgcccttgac
gactttgaccttgatatgctgcctgctgacgctcttgacgattttgaccttgacatgctccccgggtaagcgtacagcggctcccgggagtt
ctagggatctgcccctctccctcccccccccctaacgttactggccgaagccgcttggaataaggccggtgtgcgtttgtctatatgttattt
tccaccatattgccgtcttttggcaatgtgagggcccggaaacctggccctgtcttcttgacgagcattcctaggggtctttcccctctcgcc
aaaggaatgcaaggtctgttgaatgtcgtgaaggaagcagttcctctggaagcttcttgaagacaaacaacgtctgtagcgaccctttgc
aggcagcggaaccccccacctggcgacaggtgcctctgcggccaaaagccacgtgtataagatacacctgcaaaggcggcacaacc
ccagtgccacgttgtgagttggatagttgtggaaagagtcaaatggctctcctcaagcgtattcaacaaggggctgaaggatgcccaga
aggtaccccattgtatgggatctgatctggggcctcggtgcacatgctttacatgtgtttagtcgaggttaaaaaaacgtctaggccccc
gaaccacggggacgtggttttcctttgaaaaacacgatgataaggatccaccggagACTAGTGCCACCatgattgaacaaga
tggattgcacgcaggttctccggccgcttgggtggagaggctattcggctatgactgggcacaacagacaatcggctgctctgatgccg
 
ccgtgttccggctgtcagcgcaggggcgcccggttctttttgtcaagaccgacctgtccggtgccctgaatgaactgcaagacgaggca
gcgcggctatcgtggctggccacgacgggcgttccttgcgcagctgtgctcgacgttgtcactgaagcgggaagggactggctgctat
tgggcgaagtgccggggcaggatctcctgtcatctcaccttgctcctgccgagaaagtatccatcatggctgatgcaatgcggcggctg
catacgcttgatccggctacctgcccattcgaccaccaagcgaaacatcgcatcgagcgagcacgtactcggatggaagccggtcttgt
cgatcaggatgatctggacgaagagcatcaggggctcgcgccagccgaactgttcgccaggctcaaggcgagcatgcccgacggcg
aggatctcgtcgtgacccatggcgatgcctgcttgccgaatatcatggtggaaaatggccgcttttctggattcatcgactgtggccggct
gggtgtggcggaccgctatcaggacatagcgttggctacccgtgatattgctgaagagcttggcggcgaatgggctgaccgcttcctcg
tgctttacggtatcgccgctcccgattcgcagcgcatcgccttctatcgccttcttgacgagttcttctgaACTAGTCCGCGTCT
GGAACAATCAACCTCTGGATTACAAAATTTGTGAAAGATTGACTGGTATTCTTAACT
ATGTTGCTCCTTTTACGCTATGTGGATACGCTGCTTTAATGCCTTTGTATCATGCTATT
GCTTCCCGTATGGCTTTCATTTTCTCCTCCTTGTATAAATCCTGGTTGCTGTCTCTTTA
TGAGGAGTTGTGGCCCGTTGTCAGGCAACGTGGCGTGGTGTGCACTGTGTTTGCTG
ACGCAACCCCCACTGGTTGGGGCATTGCCACCACCTGTCAGCTCCTTTCCGGGACT
TTCGCTTTCCCCCTCCCTATTGCCACGGCGGAACTCATCGCCGCCTGCCTTGCCCGC
TGCTGGACAGGGGCTCGGCTGTTGGGCACTGACAATTCCGTGGTGTTGTCGGGGA
AGCTGACGTCCTTTCCATGGCTGCTCGCCTGTGTTGCCACCTGGATTCTGCGCGGG
ACGTCCTTCTGCTACGTCCCTTCGGCCCTCAATCCAGCGGACCTTCCTTCCCGCGGC
CTGCTGCCGGCTCTGCGGCCTCTTCCGCGTCTTCGCCTTCGCCCTCAGACGAGTCG
GATCTCCCTTTGGGCCGCCTCCCCGCaAAATGACCGACCAAGCGACGCCCAACCTG
CCATCACGAGATTTCGATTCCACCGCCGCCTTCTATGATCATGTCTGGGGGTGGCG
AAGAACTCCAGCATGAGATCCCCGCGCTGGAGGATCATCCAGCCGGCGTCCCGGA
AAACGATTCCGAAGCCCAACCTTTGCCTGCAGGTCGACTCTAGAGGATCCGAAAA
AACCTCCCACACCTCCCCCTGAACCTGAAACATAAAATGAATGCAATTGTTGTTGTT
AACTTGTTTATTGCAGCTTATAATGGTTACAAATAAAGCAATAGCATCACAAATTTCA
CAAATAAAGCATTTTTTTCACTGCATTCTAGTTGTGGTTTGTCCAAACTCATCAATGT
ATCTTATCATGTCTGGATCCCCGCGGCCGCGGTACCCACGTGGGCGCGCCATTTAAA
TGATAACTCAGAGAGAGTGTCCTCGAGCCAATCTGGAAGATAACCATCGGCAGCCA
TACCTGATTTAAATCATTTATTGTTCAAAGATGCAGTCATCCAAATCCACATTGACCA
GATCGCAGGCAGTGCAAGCGTCTGGCACCTTTCCCATGATATGATGAATGTAGCAC
AGTTTCTGATACGCCTTTTTGACGACAGAAACGGGTTGAGATTCTGACACGGGAAA
GCACTCTAAACAGTCTTTCTGTCCGTGAGTGAAGCAGATATTTGAATTCTGATTCAT
TCTCTCGCATTGTCTGCAGGGAAACAGCATCAGATTCATGCCCACGTGACGAGAAC
ATTTGTTTTGGTACCTGTCTGCGTAGTTGATCGAAGCTTCCGCGTCTGACGTCGATG
 
GCTGCGCAACTGACTCGCGCACCCGTTTGGGCTCACTTATATCTGCGTCACTGGGG
GCGGGTCTTTCTTGGCTCCACCCTTTTTGACGTAGAATTCATGCTCCACCTCAACC
ACGTGATCCTTTGCCCACCGGAAAAAGTCTTTGACTTCCTGCTTGGTGACCTTCCC
AAAGTCATGATCCAGACGGCGGGTGAGTTCAAATTTGAACATCCGGTCTTGCAACG
GCTGCTGGTGTTCGAAGGTCGTTGAGTTCCCGTCAATCACGGCGCACATGTTGGTG
TTGGAGGTGACGATCACGGGAGTCGGGTCTATCTGGGCCGAGGACTTGCATTTCTG
GTCCACGCGCACCTTGCTTCCTCCGAGAATGGCTTTGGCCGACTCCACGACCTTGG
CGGTCATCTTCCCCTCCTCCCACCAGATCACCATCTTGTCGACACAGTCGTTGAAG
GGAAAGTTCTCATTGGTCCAGTTTACGCACCCGTAGAAGGGCACAGTGTGGGCTAT
GGCCTCCGCGATGTTGGTCTTCCCGGTAGTTGCAGGCCCAAACAGCCAGATGGTGT
TCCTCTTGCCGAACTTTTTCGTGGCCCATCCCAGAAAGACGGAAGCCGCATATTGG
GGATCGTACCCGTTTAGTTCCAAAATTTATAAATCCGATTGCTGGAAATGTCCTCC
ACGGGCTGCTGGCCCACCAGGTAGTCGGGGGCGGTTTTAGTCAGGCTCATAATCTT
TCCCGCATTGTCCAAGGCAGCCTTGATTTGGGACCGCGAGTTGGAGGCCGCATTGA
AGGAGATGTATGAGGCCTGGTCCTCCTGGATCCACTGCTTCTCCGAGGTAATCCCCT
TGTCCACGAGCCACCCGACCAGCTCCATGTACCTGGCTGAAGTTTTTGATCTGATC
ACCGGCGCATCAGAATTGGGATTCTGATTCTCTTTGTTCTGCTCCTGCGTCTGCGAC
ACGTGCGTCAGATGCTGCGCCACCAACCGTTTACGCTCCGTGAGATTCAAACAGGC
GCTTAAATACTGTTCCATATTAGTCCACGCCCACTGGAGCTCAGGCTGGGTTTGGG
GAGCAAGTAATTGGGGATGTAGCACTCATCCACCACCTTGTTCCCGCCTCCGGCGC
CATTTCTGGTCTTTGTGACCGCGAACCAGTTTGGCAAAGTCGGCTCGATCCCGCGG
TAAATTCTCTGAATCAGTTTTTCGCGAATCTGACTCAGGAAACGTCCCAAAACCAT
GGATTTCACCCCGGTGGTTTCCACGAGCACGTGCATGTGGAAGTAGCTCTCTCCCT
TCTCAAATTGCACAAAGAAAAGGGCCTCCGGGGCCTTACTCACACGGCGCCATTCC
GTCAGAAAGTCGCGCTGCAGCTTCTCGGCCACGGTCAGGGGTGCCTGCTCAATCA
GATTCAGATCCATGTCAGAATCTGGCGGCAACTCCCATTCCTTCTCGGCCACCCAGT
TCACAAAGCTGTCAGAAATGCCGGGCAGATGCTCGTCAAGGTCGCTGGGGACCTT
AATCACAATCTCGTAAAACCCCGGCATGGCGGCGATAACTATTATCATCGTGTTTTT
CAAAGGAAAACCACGTCCCCGTGGTTCGGGGGGCCTAGACGTTTTTTAACCTCGA
CTAAACACATGTAAAGCATGTGCACCGAGGCCCCAGATCAGATCCCATACAATGGG
GTACCTTCTGGGCATCCTTCAGCCCCTTGTTGAATACGCTTGAGGAGAGCCATTTGA
CTCTTTCCACAACTATCCAACTCACAACGTGGCACTGGGGTTGTGCCGCCTTTGCA
GGTGTATCTTATACACGTGGCTTTTGGCCGCAGAGGCACCTGTCGCCAGGTGGGGG
 
GTTCCGCTGCCTGCAAAGGGTCGCTACAGACGTTGTTTGTCTTCAAGAAGCTTCCA
GAGGAACTGCTTCCTTCACGACATTCAACAGACCTTGCATTCCTTTGGCGAGAGGG
GAAAGACCCCTAGGAATGCTCGTCAAGAAGACAGGGCCAGGTTTCCGGGCCCTCA
CATTGCCAAAAGACGGCAATATGGTGGAAAATAACATATAGACAAACGCACACCGG
CCTTATTCCAAGCGGCTTCGGCCAGTAACGTTAGGGGGGGGGGCGGAATTCTATC
CCTCAGTTATCTCAGAGAGAGTGTCCTCGAGCCAATCTGGAAGATAACCATCGGCA
GCCATACCTGATTTAAATCATTTATTGTTCAAAGATGCAGTCATCCAAATCCACATTG
ACCAGATCGCAGGCAGTGCAAGCGTCTGGCACCTTTCCCATGATATGATGAATGTA
GCACAGTTTCTGATACGCCTTTTTGACGACAGAAACGGGTTGAGATTCTGACACGG
GAAAGCACTCTAAACAGTCTTTCTGTCCGTGAGTGAAGCAGATATTTGAATTCTGAT
TCATTCTCTCGCATTGTCTGCAGGGAAACAGCATCAGATTCATGCCCACGTGACGA
GAACATTGTTTTGGTACCTGTCTGCGTAGTTGATCGAAGCTTCCGCGTCTGACGTC
GATGGCTGCGCAACTGACTCGCGCACCCGTTTGGGCTCACTTATATCTGCGTCACTG
GGGGCGGGTCTTTTCTTGGCTCCACCCTTTTTGACGTAGAATTCATGCTCCACCTCA
ACCACGTGATCCTTTGCCCACCGGAAAAAGTCTTTGACTTCCTGCTTGGTGACCTT
CCCAAAGTCATGATCCAGACGGCGGGTGAGTTCAAATTTGAACATCCGGTCTTGCA
ACGGCTGCTGGTGTTCGAAGGTCGTTGAGTTCCCGTCAATCACGGCGCACATGTTG
GTGTTGGAGGTGACGATCACGGGAGTCGGGTCTATCTGGGCCGAGGACTTGCATTT
CTGGTCCACGCGCACCTTGCTTCCTCCGAGAATGGCTTTGGCCGACTCCACGACCT
TGGCGGTCATCTTCCCCTCCTCCCACCAGATCACCATCTTGTCGACACAGTCGTTGA
AGGGAAAGTTCTCATTGGTCCAGTTTACGCACCCGTAGAAGGGCACAGTGTGGGCT
ATGGCCTCCGCGATGTTGGTCTTCCCGGTAGTTGCAGGCCCAAACAGCCAGATGGT
GTTCCTCTTGCCGAACTTTTTCGTGGCCCATCCCAGAAAGACGGAAGCCGCATATT
GGGGATCGTACCCGTTTAGTTCCAAAATTTTATAAATCCGATTGCTGGAAATGTCCT
CCACGGGCTGCTGGCCCACCAGGTAGTCGGGGGCGGTTTTAGTCAGGCTCATAATC
TTTCCCGCATTGTCCAAGGCAGCCTTGATTTGGGACCGCGAGTTGGAGGCCGCATT
GAAGGAGATGTATGAGGCCTGGTCCTCCTGGATCCACTGCTTCTCCGAGGTAATCC
CCTTGTCCACGAGCCACCCGACCAGCTCCATGTACCTGGCTGAAGTTTTTGATCTGA
TCACCGGCGCATCAGAATTGGGATTCTGATTCTCTTTGTTCTGCTCCTGCGTCTGCG
ACACGTGCGTCAGATGCTGCGCCACCAACCGTTTACGCTCCGTGAGATTCAAACAG
GCGCTTAAATACTGTTCCATATTAGTCCACGCCCACTGGAGCTCAGGCTGGGTTTTG
GGGAGCAAGTAATTGGGGATGTAGCACTCATCCACCACCTTGTTCCCGCCTCCGGC
GCCATTTCTGGTCTTTGTGACCGCGAACCAGTTTGGCAAAGTCGGCTCGATCCCGC
 
GGTAAATTCTCTGAATCAGTTTTCGCGAATCTGACTCAGGAAACGTCCCAAAACC
ATGGATTTCACCCCGGTGGTTTCCACGAGCACGTGCATGTGGAAGTAGCTCTCTCC
CTTCTCAAATTGCACAAAGAAAAGGCCTCCGGGGCCTTACTCACACGGCGCCATT
CCGTCAGAAAGTCGCGCTGCAGCTTCTCGGCCACGGTCAGGGGTGCCTGCTCAAT
CAGATTCAGATCCATGTCAGAATCTGGCGGCAACTCCCATTCCTTCTCGGCCACCCA
GTTCACAAAGCTGTCAGAAATGCCGGGCAGATGCTCGTCAAGGTCGCTGGGGACC
TTAATCACAATCTCGTAAAACCCCGGCATGGCGGCGATAACTATTATCATCGTGTTTT
TCAAAGGAAAACCACGTCCCCGTGGTTCGGGGGGCCTAGACGTTTTTTAACCTCG
ACTAAACACATGTAAAGCATGTGCACCGAGGCCCCAGATCAGATCCCATACAATGG
GGTACCTTCTGGGCATCCTTCAGCCCCTTGTTGAATACGCTTGAGGAGAGCCATTTG
ACTCTTTCCACAACTATCCAACTCACAACGTGGCACTGGGGTTGTGCCGCCTTTGC
AGGTGTATCTTATACACGTGGCTTTTGGCCGCAGAGGCACCTGTCGCCAGGTGGGG
GGTTCCGCTGCCTGCAAAGGGTCGCTACAGACGTTGTTTGTCTTCAAGAAGCTTCC
AGAGGAACTGCTTCCTTCACGACATTCAACAGACCTTGCATTCCTTTGGCGAGAGG
GGAAAGACCCCTAGGAATGCTCGTCAAGAAGACAGGGCCAGGTTTCCGGGCCCTC
ACATTGCCAAAAGACGGCAATATGGTGGAAAATAACATATAGACAAACGCACACCG
GCCTTATTCCAAGCGGCTTCGGCCAGTAACGTTAGGGGGGGGGGCGGAATTCTAT
CCCTCAGTTATCTCAGAGAGAGTGTCCTCGAGCCAATCTGGAAGATAACCATCGGC
AGCCATACCTGATTTAAATCATTTATTGTTCAAAGATGCAGTCATCCAAATCCACATT
GACCAGATCGCAGGCAGTGCAAGCGTCTGGCACCTTTCCCATGATATGATGAATGT
AGCACAGTTTCTGATACGCCTTTTTGACGACAGAAACGGGTTGAGATTCTGACACG
GGAAAGCACTCTAAACAGTCTTTCTGTCCGTGAGTGAAGCAGATATTTGAATTCTG
ATTCATTCTCTCGCATTGTCTGCAGGGAAACAGCATCAGATTCATGCCCACGTGACG
AGAACATTTGTTTTGGTACCTGTCTGCGTAGTTGATCGAAGCTTCCGCGTCTGACGT
CGATGGCTGCGCAACTGACTCGCGCACCCGTTTGGGCTCACTTATATCTGCGTCACT
GGGGGCGGGTCTTTTCTTGGCTCCACCCTTTTTGACGTAGAATTCATGCTCCACCTC
AACCACGTGATCCTTTGCCCACCGGAAAAAGTCTTTGACTTCCTGCTTGGTGACCT
TCCCAAAGTCATGATCCAGACGGCGGGTGAGTTCAAATTTGAACATCCGGTCTTGC
AACGGCTGCTGGTGTTCGAAGGTCGTTGAGTTCCCGTCAATCACGGCGCACATGTT
GGTGTTGGAGGTGACGATCACGGGAGTCGGGTCTATCTGGGCCGAGGACTTGCATT
TCTGGTCCACGCGCACCTTGCTTCCTCCGAGAATGGCTTTGGCCGACTCCACGACC
TTGGCGGTCATCTTCCCCTCCTCCCACCAGATCACCATCTTGTCGACACAGTCGTTG
AAGGGAAAGTTCTCATTGGTCCAGTTTACGCACCCGTAGAAGGGCACAGTGTGGG
 
CTATGGCCTCCGCGATGTTGGTCTTCCCGGTAGTTGCAGGCCCAAACAGCCAGATG
GTGTTCCTCTTGCCGAACTTTTCGTGGCCCATCCCAGAAAGACGGAAGCCGCATA
TTGGGGATCGTACCCGTTTAGTTCCAAAATTTATAAATCCGATTGCTGGAAATGTC
CTCCACGGGCTGCTGGCCCACCAGGTAGTCGGGGGCGGTTTAGTCAGGCTCATAA
TCTTTCCCGCATTGTCCAAGGCAGCCTTGATTTGGGACCGCGAGTTGGAGGCCGCA
TTGAAGGAGATGTATGAGGCCTGGTCCTCCTGGATCCACTGCTTCTCCGAGGTAATC
CCCTTGTCCACGAGCCACCCGACCAGCTCCATGGTGGCGTTATCCGGCCGATCGAT
AGATCTGGGCCCCGGTGTCGACTTTACGAGGGTAGGAAGTGGTACGGAAAGTTGG
TATAAGACAAAAGTGTTGTGGAATTGCTGGAGGCGATCTGACGGTTCACTAAACGA
GCTCTGCTTTATAGGCGCCCACCGTACACGCCTAAAGCTTATACGTTCTCTATCACT
GATAGGGAGTAAACTGGATATACGTTCTCTATCACTGATAGGGAGTAAACTGTAGAT
ACGTTCTCTATCACTGATAGGGAGTAAACTGGATATACGTTCTCTATCACTGATAGG
GAGTAAACTCCTTATACGTTCTCTATCACTGATAGGGAGTAAAGTCTGCATACGTTC
TCTATCACTGATAGGGAGTAAACTCTTCATACGTTCTCTATCACTGATAGGGAGTAA
ACgaatggCTAggATCCGGCCGGccTGCAggTGTCCTCACAGGAACGAAGTCCCTAAAG
AAACAGTGGCAGCCAGGTTTAGCCCCGGAATTGACTGGATTCCTTTTTTAGGGCCC
ATTGGTATGGCTTTTTCCCCGTATCCCCCCAGGTGTCTGCAGGCTCAAAGAGCAGC
GAGAAGCGTTCAGAGGAAAGCGATCCCGTGCCACCTTCCCCGTGCCCGGGCTGTC
CCCGCACGCTGCCGGCTCGGGGATGCGGGGGGAGCGCCGGACCGGAGCGGAGCC
CCGGGCGGCTCGCTGCTGCCCCCTAGCGGGGGAGGGACGTAATTACATCCCTGGGG
GCTTTGGGGGGGGGCTGTCCCTCTAGAGCGGCCGCCACCGCGGTGGAGCTCCAGC
TTTTGTTCGCGAATTGGGTACCGGGCCCCCCCTCGAGGTCGACGGTATCGGCTAGCT
AGGGATAACAGGGTAATCCTCCAAGGCCAGCTTCCCACAATAAGTTGGGTGAATTT
TGGCTCATTCCTCCTTTCTATAGGATTGAGGTCAGAGCTTTGTGATGGGAATTCTGT
GGAATGTGTGTCAGTTAGGGTGTGGAAAGTCCCgcGATCAAACGCCAGCAACGCGG
CCTTTTTACGGTTCCTGGCCTTTTGCTGGCCTTTTGCTCACATGTCCTGCAGGCAGC
TGCGCGCTCGCTCGCTCACTGAGGCCGCCCGGGCAAAGCCCGGGCGTCGGGCGAC
CTTTGGTCGCCCGGCCTCAGTGAGCGAGCGAGCGCGCAGAGAGGGAGTGGCCAA
CTCCATCACTAGGGGTTCCTGCGGCCGCACGCGTGGAGCTAGTTATTAATAGTAATC
AATTACGGGGTCATTAGTTCATAGCCCATATATGGAGTTCCGCGTTACATAACTTACG
GTAAATGGCCCGCCTGGCTGACCGCCCAACGACCCCCGCCCATTGACGTCAATAAT
GACGTATGTTCCCATAGTAACGTCAATAGGGACTTTCCATTGACGTCAATGGGTGGA
GTATTTACGGTAAACTGCCCACTTGGCAGTACATCAAGTGTATCATATGCCAAGTAC
 
GCCCCCTATTGACGTCAATGACGGTAAATGGCCCGCCTGGCATTATGCCCAGTACAT
GACCTTATGGGACTTTCCTACTTGGCAGTACATCTACGTATTAGTCATCGCTATTACC
ATGGTGATGCGGTTTTGGCAGTACATCAATGGGCGTGGATAGCGGTTTGACTCACG
GGGATTTCCAAGTCTCCACCCCATTGACGTCAATGGGAGTTTGTTTTGCACCAAAA
TCAACGGGACTTTCCAAAATGTCGTAACAACTCCGCCCCATTGACGCAAATGGGCG
GTAGGCGTGTACGGTGGGAGGTCTATATAAGCAGAGCTCGTTTAGTGAACCGTCAG
ATCGCCTGGAGACGCCATCCACGCTGTTTTGACCTCCATAGAAGACACCGGGACCG
ATCCAGCCTCCGCGGATTCGAATCCCGGCCGGGAACGGTGCATTGGAACGCGGATT
CCCCGTGCCAAGAGTGACGTAAGTACCGCCTATAGAGTCTATAGGCCCACAAAAAA
TGCTTTCTTCTTTTAATATACTTTTTTGTTTATCTTATTTCTAATACTTTCCCTAATCTC
TTTCTTTCAGGGCAATAATGATACAATGTATCATGCCTCTTTGCACCATTCTAAAGAA
TAACAGTGATAATTTCTGGGTTAAGGCAATAGCAATATTTCTGCATATAAATATTTCT
GCATATAAATTGTAACTGATGTAAGAGGTTTCATATTGCTAATAGCAGCTACAATCCA
GCTACCATTCTGCTTTTATTTTATGGTTGGGATAAGGCTGGATTATTCTGAGTCCAAG
CTAGGCCCTTTTGCTAATCATGTTCATACCTCTTATCTTCCTCCCACAGCTCCTGGGC
AACGTGCTGGTCTGTGTGCTGGCCCATCACTTTGGCAAAGAATTGGGATTCGAACA
TCGATTGAATTCTGAATGGTGAGCAAGGGCGAGGAGCTGTTCACCGGGGTGGTGC
CCATCCTGGTCGAGCTGGACGGCGACGTAAACGGCCACAAGTTCAGCGTGTCCGG
CGAGGGCGAGGGCGATGCCACCTACGGCAAGCTGACCCTGAAGTTCATCTGCACC
ACCGGCAAGCTGCCCGTGCCCTGGCCCACCCT
 
CGTGACCACCCTGACCTACGGCGTGCAGTGCTTCAGCCGCTACCCCGACC
ACATGAAGCAGCACGACTTCTTCAAGTCCGCCATGCCCGAAGGCTACGTCCAGGA
GCGCACCATCTTCTTCAAGGACGACGGCAACTACAAGACCCGCGCCGAGGTGAAG
TTCGAGGGCGACACCCTGGTGAACCGCATCGAGCTGAAGGGCATCGACTTCAAGG
AGGACGGCAACATCCTGGGGCACAAGCTGGAGTACAACTACAACAGCCACAACGT
CTATATCATGGCCGACAAGCAGAAGAACGGCATCAAGGTGAACTTCAAGATCCGCC
ACAACATCGAGGACGGCAGCGTGCAGCTCGCCGACCACTACCAGCAGAACACCCC
CATCGGCGACGGCCCCGTGCTGCTGCCCGACAACCACTACCTGAGCACCCAGTCC
GCCCTGAGCAAAGACCCCAACGAGAAGCGCGATCACATGGTCCTGCTGGAGTTCG
TGACCGCCGCCGGGATCACTCTCGGCATGGACGAGCTGTACAAGTACTCAGATCTC
GAGCTCAAGTAGGGATCCTCTAGAGTCGACCTGCAGAAGCTTGCCTCGAGCAGCG
CTGCTCGAGAGATCTACGGGTGGCATCCCTGTGACCCCTCCCCAGTGCCTCTCCTG
GCCCTGGAAGTTGCCACTCCAGTGCCCACCAGCCTTGTCCTAATAAAATTAAGTTG
 
CATCATTTTGTCTGACTAGGTGTCCTTCTATAATATTATGGGGTGGAGGGGGGTGGTA
TGGAGCAAGGGGCAAGTTGGGAAGACAACCTGTAGGGCCTGCGGGGTCTATTGGG
AACCAAGCTGGAGTGCAGTGGCACAATCTTGGCTCACTGCAATCTCCGCCTCCTGG
GTTCAAGCGATTCTCCTGCCTCAGCCTCCCGAGTTGTTGGGATTCCAGGCATGCATG
ACCAGGCTCAGCTAATTTTTGTTTTTTTGGTAGAGACGGGGTTTCACCATATTGGCC
AGGCTGGTCTCCAACTCCTAATCTCAGGTGATCTACCCACCTTGGCCTCCCAAATTG
CTGGGATTACAGGCGTGAACCACTGCTCCCTTCCCTGTCCTTCTGATTTTGTAGGTA
ACCACGTGCGGACCGAGCGGCCGCAGGAACCCCTAGTGATGGAGTTGGCCACTCC
CTCTCTGCGCGCTCGCTCGCTCACTGAGGCCGGGCGACCAAAGGTCGCCCGACGC
CCGGGCTTTGCCCGGGCGGCCTCAGTGAGCGAGCGAGCGCGCAGCTGCCTGCAGG
GGCGCCTGATGCGGTATTTTCTCCTTACGCATCTGTGCGGTATTTCACACCGCATAC
GTCgtaGCtGATcaATTgGCGCGCCGAATTCAAGCTaCGCgtAcgATAAGCTTGATATCTAT
AACAAGAAAATATATATATAATAAGTTATCACGTAAGTAGAACATGAAATAACAATAT
AATTATCGTATGAGTTAAATATTACCCTGTTATCCCTAGCTAGCTCGCTACCTTAGGA
CCGTTATAGTTACTTAAAAGTCACGTAAAAGATAATCATGCGTCATTTTGACTCACG
CGGTCGTTATAGTTCAAAATCAGTGACACTTACCGCATTGACAAGCACGCCTCACG
GGAGCTCCAAGCGGCGACTGAGATGTCCTAAATGCACAGCGACGGATTCGCGCTAT
TTAGAAAGAGAGAGCAATATTTCAAGAATGCATGCGTCAATTTTACGCAGACTATCT
TTCTAGGGTTAATCTAGCTGCATCAGGATCATATCGTCGGGTCTTTTTTCCGGCTCAG
TCATCGCCCAAGCTGGCGCTATCTGGGCATCGGGGAGGAAGAAGCCCGTGCCTTTT
CCCGCGAGGTTGAAGCGGCATGGAAAGAGTTTGCCGAGGATGACTGCTGCTGCAT
TGACGTTGAGCGAAAACGCACGTTTACCATGATGATTCGGGAAGGTGTGGCCATGC
ACGCCTTTAACGGTGAACTGTTCGTTCAGGCCACCTGGGATACCAGTTCGTCGCGG
CTTTTCCGGACACAGTTCCGGATGGTCAGCCCGAAGCGCATCAGCAACCCGAACA
ATACCGGCGACAGCCGGAACTGCCGTGCCGGTGTGCAGATTAATGACAGCGGTGC
GGCGCTGGGATATTACGTCAGCGAGGACGGGTATCCTGGCTGGATGCCGCAGAAAT
GGACATGGATACCCCGTGAGTTACCCGGCGGGCGCGCTTGGCGTAATCATGGTCAT
AGCTGTTTCCTGTGTGAAAATTGCGTTGCGCTCACTGCCCGCTTTCCAGTCGGGAA
ACCTGTCGTGCCAGCTGCATTAATGAATCGGCCAACGCGCGGGGAGAGGCGGTTTG
CGTATTGGGCGCTCTTCCGCTTCCTCGCTCACTGACTCGCTGCGCTCGGTCGTTCGG
CTGCGGCGAGCGGTATCAGCTCACTCAAAGGCGGTAATACGGTTATCCACAGAATC
AGGGGATAACGCAGGAAAGAACATGTGAGCAAAAGGCCAGCAAAAGGCCAGGAA
CCGTAAAAAGGCCGCGTTGCTGGCGTT
SEQ ID NO: 2: 
gaattcgagcttgcatgcctgcaggtcgttacataacttacggtaaatggcccgcctggctgaccgcccaacgacccccgc
ccattgacgtcaataatgacgtatgttcccatagtaacgccaatagggactttccattgacgtcaatgggtggagtatttacggtaaactgc
ccacttggcagtacatcaagtgtatcatatgccaagtacgccccctattgacgtcaatgacggtaaatggcccgcctggcattatgcccag
tacatgaccttatgggactttcctacttggcagtacatctacgtattagtcatcgctattaccatggtgatgcggttttggcagtacatcaatgg
gcgtggatagcggtttgactcacggggatttccaagtctccaccccattgacgtcaatgggagtttgttttggcaccaaaatcaacgggac
tttccaaaatgtcgtaacaactccgccccattgacgcaaatgggcggtaggcgtgtacggtgggaggtctatataagcagagctcgttta
gtgaaccgtcagatcgcctggagacgccatccacgctgttttgacctccatagaagacaccgggaccgatccagcctccggactctag
aggatccggtactcgaggaactgaaaaaccagaaagttaactggtaagtttagtctttttgtcttttatttcaggtcccggatccggtggtgg
tgcaaatcaaagaactgctcctcagtggatgttgcctttacttctaggcctgtacggaagtgttacttctgctctaaaagctgcggaattgta
cccgcggataaaatgggtagttctttagacgatgagcatatcctctctgctcttctgcaaagcgatgacgagcttgttggtgaggattctga
cagtgaaatatcagatcacgtaagtgaagatgacgtccagagcgatacagaagaagcgtttatagatgaggtacatgaagtgcagccaa
cgtcaagcggtagtgaaatattagacgaacaaaatgttattgaacaaccaggttcttcattggcttctaacagaatcttgaccttgccacag
aggactattagaggtaagaataaacattgttggtcaacttcaaagtccacgaggcgtagccgagtctctgcactgaacattgtcagatctc
aaagaggtccgacgcgtatgtgccgcaatatatatgacccacttttatgcttcaaactatttttactgatgagataatttcggaaattgtaaaa
tggacaaatgctgagatatcattgaaacgtcgggaatctatgacaggtgctacatttcgtgacacgaatgaagatgaaatctatgctttcttt
ggtattctggtaatgacagcagtgagaaaagataaccacatgtccacagatgacctctttgatcgatctttgtcaatggtgtacgtctctgta
atgagtcgtgatcgttttgatttttgatacgatgtcttagaatggatgacaaaagtatacggcccacacttcgagaaaacgatgtatttactc
ctgttagaaaaatatgggatctctttatccatcagtgcatacaaaattacactccaggggctcatttgaccatagatgaacagttacttggtttt
agaggacggtgtccgtttaggatgtatatcccaaacaagccaagtaagtatggaataaaaatcctcatgatgtgtgacagtggtacgaag
tatatgataaatggaatgccttatttgggaagaggaacacagaccaacggagtaccactcggtgaatactacgtgaaggagttatcaaag
cctgtgcacggtagttgtcgtaatattacgtgtgacaattggttcacctcaatccctttggcaaaaaacttactacaagaaccgtataagttaa
ccattgtgggaaccgtgcgatcaaacaaacgcgagataccggaagtactgaaaaacagtcgctccaggccagtgggaacatcgatgtt
ttgttttgacggaccccttactctcgtctcatataaaccgaagccagctaagatggtatacttattatcatcttgtgatgaggatgcttctatca
acgaaagtaccggtaaaccgcaaatggttatgtattataatcaaactaaaggcggagtggacacgctagaccaaatgtgttctgtgatga
cctgcagtaggaagacgaataggtggcctatggcattattgtacggaatgataaacattgcctgcataaattcttttattatatacagccata
atgtcagtagcaagggagaaaaggttcaaagtcgcaaaaaatttatgagaaacctttacatgagcctgacgtcatcgtttatgcgtaagcg
tttagaagctcctactttgaagagatatttgcgcgataatatctctaatattttgccaaatgaagtgcctggtacatcagatgacagtactgaa
gagccagtaatgaaaaaacgtacttactgtacttactgcccctctaaaataaggcgaaaggcaaatgcatcgtgcaaaaaatgcaaaaaa
gttatttgtcgagagcataatattgatatgtgccaaagttgtttctgactgactaataagtataatttgtttctattatgtataagttaagctaatta
ggatctaagctgcaataaacaagttaacaacaacaattgcattcattttatgtttcaggttcagggggaggtgtgggaggttttttcggatcc
tctagagtcgacctgcaggcatgcaagcttggcgtaatcatggtcatagctgtttcctgtgtgaaattgttatccgctcacaattccacacaa
catacgagccggaagcataaagtgtaaagcctggggtgcctaatgagtgagctaactcacattaattgcgttgcgctcactgcccgctttc
 
cagtcgggaaacctgtcgtgccagctgcattaatgaatcggccaacgcgcggggagaggcggtttgcgtattgggcgctcttccgcttc
ctcgctcactgactcgctgcgctcggtcgttcggctgcggcgagcggtatcagctcactcaaaggcggtaatacggttatccacagaatc
aggggataacgcaggaaagaacatgtgagcaaaaggccagcaaaaggccaggaaccgtaaaaaggccgcgttgctggcgtttttcc
ataggctccgcccccctgacgagcatcacaaaaatcgacgctcaagtcagaggtggcgaaacccgacaggactataaagataccagg
cgtttccccctggaagctccctcgtgcgctctcctgttccgaccctgccgcttaccggatacctgtccgcctttctcccttcgggaagcgtg
gcgctttctcatagctcacgctgtaggtatctcagttcggtgtaggtcgttcgctccaagctgggctgtgtgcacgaaccccccgttcagc
ccgaccgctgcgccttatccggtaactatcgtcttgagtccaacccggtaagacacgacttatcgccactggcagcagccactggtaac
aggattagcagagcgaggtatgtaggcggtgctacagagttcttgaagtggtggcctaactacggctacactagaagaacagtatttggt
atctgcgctctgctgaagccagttaccttcggaaaaagagttggtagctcttgatccggcaaacaaaccaccgctggtagcggtggtttttt
tgtttgcaagcagcagattacgcgcagaaaaaaaggatctcaagaagatcctttgatcttttctacggggtctgacgctcagtggaacgaa
aactcacgttaagggattttggtcatgagattatcaaaaaggatcttcacctagatccttttaaattaaaaatgaagttttaaatcaatctaaag
tatatatgagtaaacttggtctgacagttaccaatgcttaatcagtgaggcacctatctcagcgatctgtctatttcgttcatccatagttgcct
gactccccgtcgtgtagataactacgatacgggagggcttaccatctggccccagtgctgcaatgataccgcgagacccacgctcacc
ggctccagatttatcagcaataaaccagccagccggaagggccgagcgcagaagtggtcctgcaactttatccgcctccatccagtcta
ttaattgttgccgggaagctagagtaagtagttcgccagttaatagtttgcgcaacgttgttgccattgctacaggcatcgtggtgtcacgct
cgtcgtttggtatggcttcattcagctccggttcccaacgatcaaggcgagttacatgatcccccatgttgtgcaaaaaagcggttagctcc
ttcggtcctccgatcgttgtcagaagtaagttggccgcagtgttatcactcatggttatggcagcactgcataattctcttactgtcatgccat
ccgtaagatgcttttctgtgactggtgagtactcaaccaagtcattctgagaatagtgtatgcggcgaccgagttgctcttgcccggcgtca
atacgggataataccgcgccacatagcagaactttaaaagtgctcatcattggaaaacgttcttcggggcgaaaactctcaaggatcttac
cgctgttgagatccagttcgatgtaacccactcgtgcacccaactgatcttcagcatcttttactttcaccagcgtttctgggtgagcaaaaa
caggaaggcaaaatgccgcaaaaaagggaataagggcgacacggaaatgttgaatactcatactcttcctttttcaatattattgaagcat
ttatcagggttattgtctcatgagcggatacatatttgaatgtatttagaaaaataaacaaataggggttccgcgcacatttccccgaaaagt
gccacctgacgtctaagaaaccattattatcatgacattaacctataaaaataggcgtatcacgaggccctttcgtctcgcgcgtttcggtg
atgacggtgaaaacctctgacacatgcagctcccggagacggtcacagcttgtctgtaagcggatgccgggagcagacaagcccgtc
agggcgcgtcagcgggtgttggcgggtgtcggggctggcttaactatgcggcatcagagcagattgtactgagagtgcaccatatgcg
gtgtgaaataccgcacagatgcgtaaggagaaaataccgcatcaggcgccattcgccattcaggctgcgcaactgttgggaagggcg
atcggtgcgggcctcttcgctattacgccagctggcgaaagggggatgtgctgcaaggcgattaagttgggtaacgccagggttttccc
agtcacgacgttgtaaaacgacggccagt
SEQ ID NO: 3: GGAACCCCTA GTGATGGAGT T
SEQ ID NO: 4: CGGCCTCAGT GAGCGA
SEQ ID NO: 5: CACTCCCTCT CTGCGCGCTC G
[Sequence Table] International application 23F01287W1JP24046425_154.xml based on International Patent Cooperation Treaty

## Claims

1. A method for producing a viral vector, the method comprising:
culturing producer cells or packaging cells in a culture solution containing the producer cells or the packaging cells at a cell density of 1 × 10⁶ cells/mL or more and a histone deacetylase inhibitor.

2. The production method according to claim 1,
wherein the histone deacetylase inhibitor is a compound having a benzamide structure or a hydroxamic acid.

3. The production method according to claim 1,
wherein the histone deacetylase inhibitor is a compound represented by Formula (1) or Formula (2),
R¹-CONH-R² (1)
in Formula (1), one of R¹ or R² is an aromatic group which may have a substituent, and the other of R¹ or R² is an amino group which may have a substituent, an alkyl group which may have a substituent, an alkenyl group which may have a substituent, an aromatic group which may have a substituent, or an alkoxy group which may have a substituent,
R³-CONH-OH (2)
in Formula (2), R³ is an alkyl group which may have a substituent or an alkenyl group which may have a substituent.

4. The production method according to claim 1,
wherein the histone deacetylase inhibitor is a compound represented by Formula (1A), Formula (1B), or Formula (2A), 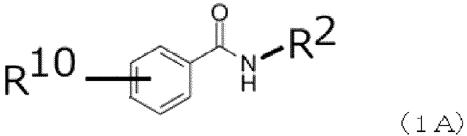
in Formula (1A), R¹⁰ represents halogen, a heterocyclic group which may have a substituent, an alkyl group having 1 to 6 carbon atoms which may have a substituent, or an amino group which may have a substituent, and R² is an amino group which may have a substituent, an alkyl group which may have a substituent, or an aromatic group which may have a substituent, 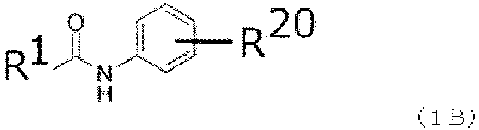
in Formula (1B), R²⁰ represents an amino group, and R¹ is an alkyl group which may have a substituent or an aromatic group which may have a substituent,
R³¹-R³⁰-CONH-OH (2A)
in Formula (2A), R³⁰ represents -(CH₂)ₙ- or CH=CH-, n represents an integer of 2 to 8, and R³¹ represents an aromatic group which may have a substituent or CONH-OH.

5. The production method according to claim 4,
wherein in Formula (1A), R¹⁰ is
bromine, 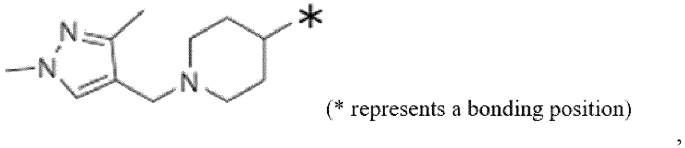
methyl, 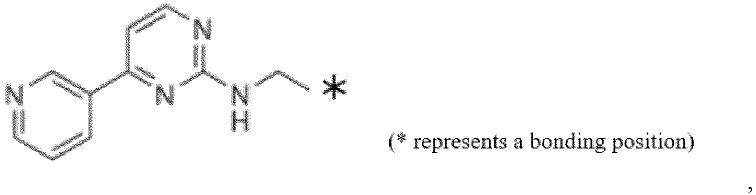 or
CH₃-CO-NH-.

6. The production method according to claim 1,
wherein the histone deacetylase inhibitor is any one of the following compounds,

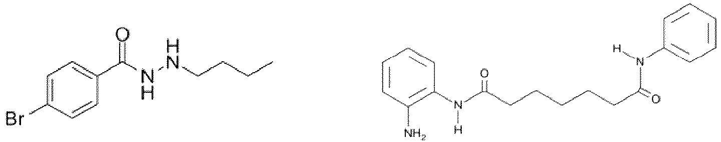

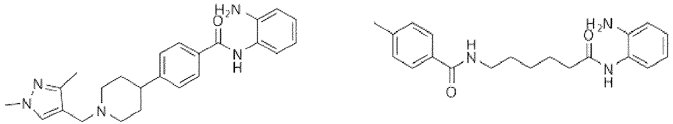

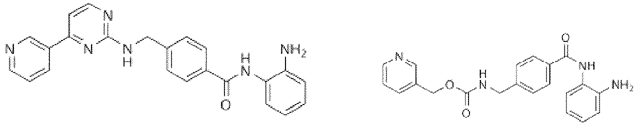

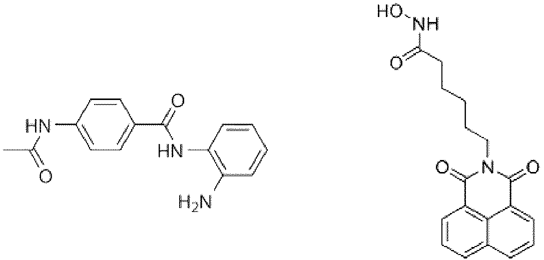

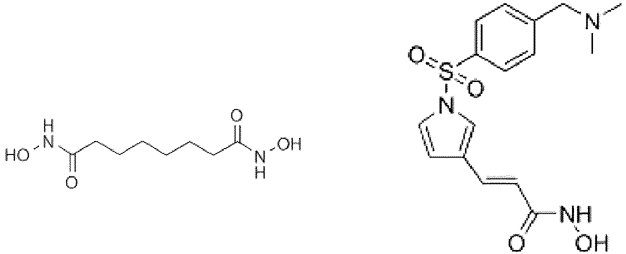

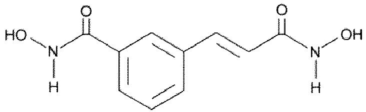

7. The production method according to any one of claims 1 to 6, comprising:
culturing the cells by perfusion culture.

8. The production method according to any one of claims 1 to 6,
wherein a virus is an adeno-associated virus.

9. The production method according to claim 8,
wherein the producer cells or the packaging cells are cells in which a Rep gene, a Cap gene, and a helper gene are incorporated into a chromosome.

10. The production method according to any one of claims 1 to 6,
wherein the cells are animal cells.

11. The production method according to any one of claims 1 to 6,
wherein a content of the histone deacetylase inhibitor in the culture solution is 50 µmol/L or less.

12. The production method according to any one of claims 1 to 6,
wherein the culture is performed for 24 hours or more in the culture solution containing the histone deacetylase inhibitor.

13. The production method according to any one of claims 1 to 6,
wherein the cell density is 30 × 10⁶ cells/mL or more.

14. The production method according to any one of claims 1 to 6,
wherein the culture is a suspension culture.
